# EUROPEAN PATENT APPLICATION

(11) **EP 4 579 220 A1**
(43) Date of publication of application: **02.07.2025**
(21) Application number: 23871783.9
(22) Date of filing: 06.09.2023
(51) Int. Cl.: G01N 21/65, C12M 1/00, C12M 1/34, C12P 21/02, G01N 30/02, G01N 30/86, G01N 30/88

(54) **INFORMATION PROCESSING DEVICE, OPERATION METHOD OF INFORMATION PROCESSING DEVICE, OPERATION PROGRAM OF INFORMATION PROCESSING DEVICE, AND STATE PREDICTION MODEL**

(30) Priority: 27.09.2022 JP 2022154075
(71) Applicant: FUJIFILM Corporation, Tokyo 106-8620 (JP)
(72) Inventor: SUGITA, Yui, Ashigarakami-gun, Kanagawa 258-8577 (JP)
(74) Representative: Hoffmann Eitle
(86) International application number: PCT/JP2023/032535
(87) International publication number: WO 2024/070543

(57) **Abstract**

An information processing device includes a processor, in which the processor is configured to: as preparatory processing for generating a state prediction model that predicts a state of a target component in a suspension produced in a manufacturing process of a biopharmaceutical containing a target protein as an active ingredient, acquire first spectrum measurement data obtained by measuring a spectrum of an electromagnetic wave emitted from the target protein and second spectrum measurement data obtained by measuring a spectrum of an electromagnetic wave emitted from the target component; and select a specific wave number band or a specific wavelength band that is specific to the target component by comparing an intensity value of the first spectrum measurement data and an intensity value of the second spectrum measurement data.

## Description

### BACKGROUND OF THE INVENTION

### 1. Field of the Invention

The present disclosed technology relates to an information processing device, an operation method of an information processing device, an operation program of an information processing device, and a state prediction model.

### 2. Description of the Related Art

A manufacturing process for a biopharmaceutical in which a target protein, such as an antibody, is an active ingredient is known. In such a manufacturing process, a suspension in which various components including the target protein are dispersed in a liquid is often produced. Monitoring a state of the target component in the suspension is important for determining whether the manufacturing process is successful or failed.

JP2016-128822A discloses a technology of predicting a concentration of an aggregate of a target protein as a state of a target component. Specifically, in JP2016-128822A, the concentration of the aggregate is predicted from spectrum measurement data obtained by measuring a Raman spectrum of a suspension using a linear model such as a partial least squares (PLS) model.

### SUMMARY OF THE INVENTION

In the technology disclosed in JP2016-128822A, the prediction accuracy of the concentration of the aggregate is not so high, and the practicality is poor. The reason for this is considered to be that a wave number band, which is considered to contribute to the prediction of the concentration of the aggregate, is not selected from among the wave numbers of the Raman spectrum measurement data.

As a method of selecting the wave number band, which is considered to contribute to the prediction of the concentration of the aggregate, for example, sparse modeling can be considered. However, the wave number band selected by the sparse modeling is highly dependent on the Raman spectrum measurement data prepared for the selection. Therefore, it cannot be said that the wave number band selected by the sparse modeling is a reasonable wave number band, which is thought to truly contribute to the prediction of the concentration of the aggregate.

One embodiment according to the present disclosed technology provides an information processing device, an operation method of an information processing device, and an operation program of an information processing device, with which a reasonable wave number band or wavelength band of spectrum measurement data can be selected, which is considered to contribute to prediction of a state of a target component in a suspension produced in a manufacturing process of a biopharmaceutical.

In addition, one embodiment according to the present disclosed technology provides a state prediction model with which a state of a target component in a suspension produced in a manufacturing process of a biopharmaceutical can be predicted with higher accuracy than in the related art.

The present disclosure provides an information processing device comprising: a processor, in which the processor is configured to: as preparatory processing for generating a state prediction model that predicts a state of a target component in a suspension produced in a manufacturing process of a biopharmaceutical containing a target protein as an active ingredient, acquire first spectrum measurement data obtained by measuring a spectrum of an electromagnetic wave emitted from the target protein and second spectrum measurement data obtained by measuring a spectrum of an electromagnetic wave emitted from the target component; and select a specific wave number band or a specific wavelength band that is specific to the target component by comparing an intensity value of the first spectrum measurement data and an intensity value of the second spectrum measurement data.

It is preferable that the state prediction model is generated by using a data set including an intensity value of the specific wave number band or the specific wavelength band and ground truth data of the state of the target component.

It is preferable that the state of the target component is a concentration of the target component in the suspension, and a concentration of the target protein and the concentration of the target component in the suspension as a source of the data set are in a range of 0.001 mg/mL to 20 mg/mL.

It is preferable that a suspension to be used to select the specific wave number band or the specific wavelength band is subjected to a pretreatment for promoting generation of the target component.

It is preferable that the state prediction model outputs a prediction result of the state of the target component in accordance with an intensity value of the specific wave number band or the specific wavelength band in third spectrum measurement data obtained by measuring a spectrum of an electromagnetic wave emitted from a suspension in which the state of the target component is unknown.

It is preferable that the third spectrum measurement data is data measured during progress of the manufacturing process.

It is preferable that the third spectrum measurement data is data measured after a virus inactivation treatment or after a cation chromatography treatment.

It is preferable that the first spectrum measurement data and the second spectrum measurement data are data measured from a first solution containing the target protein and a second solution containing the target component, the first solution and the second solution being separated from the suspension by using a high-performance liquid chromatography device.

It is preferable that the target component is an aggregate of the target protein.

It is preferable that the state prediction model is a machine learning model.

It is preferable that the target protein is an antibody.

It is preferable that the spectrum is a Raman spectrum.

It is preferable that the specific wave number band is in at least any one of a range of 1220 cm⁻¹ to 1260 cm⁻¹ or a range of 1650 cm⁻¹ to 1690 cm⁻¹.

The present disclosure provides an operation method of an information processing device, the operation method comprising: as preparatory processing for generating a state prediction model that predicts a state of a target component in a suspension produced in a manufacturing process of a biopharmaceutical containing a target protein as an active ingredient, acquiring first spectrum measurement data obtained by measuring a spectrum of an electromagnetic wave emitted from the target protein and second spectrum measurement data obtained by measuring a spectrum of an electromagnetic wave emitted from the target component; and selecting a specific wave number band or a specific wavelength band that is specific to the target component by comparing an intensity value of the first spectrum measurement data and an intensity value of the second spectrum measurement data.

The present disclosure provides an operation program of an information processing device, the operation program causing a computer to execute a process comprising: as preparatory processing for generating a state prediction model that predicts a state of a target component in a suspension produced in a manufacturing process of a biopharmaceutical containing a target protein as an active ingredient, acquiring first spectrum measurement data obtained by measuring a spectrum of an electromagnetic wave emitted from the target protein and second spectrum measurement data obtained by measuring a spectrum of an electromagnetic wave emitted from the target component; and selecting a specific wave number band or a specific wavelength band that is specific to the target component by comparing an intensity value of the first spectrum measurement data and an intensity value of the second spectrum measurement data.

The present disclosure provides a state prediction model causing a computer to execute a function comprising: outputting a prediction result of a state of a target component in a suspension produced in a manufacturing process of a biopharmaceutical containing a target protein as an active ingredient, in accordance with an intensity value of a specific wave number band or a specific wavelength band that is specific to the target component among intensity values of wave numbers or wavelengths of spectrum measurement data obtained by measuring a spectrum of an electromagnetic wave emitted from the suspension.

According to the present disclosed technology, it is possible to provide the information processing device, the operation method of the information processing device, and the operation program of the information processing device, with which the intensity value of the reasonable wave number band or wavelength band of the spectrum measurement data can be selected, which is considered to contribute to the prediction of the state of the target component in the suspension produced in the manufacturing process of the biopharmaceutical.

In addition, according to the present disclosed technology, it is possible to provide the state prediction model with which the state of the target component in the suspension produced in the manufacturing process of the biopharmaceutical can be predicted with higher accuracy than in the related art.

### BRIEF DESCRIPTION OF THE DRAWINGS

Fig. 1 is a diagram showing an outline of a manufacturing process of a biopharmaceutical.
Fig. 2 is a diagram showing an information processing system.
Fig. 3 is a block diagram of computers constituting a selection device, a learning device, and an operation device.
Fig. 4 is a diagram showing a pretreatment performed on a second purified solution, a high-performance liquid chromatography device, and data input to the selection device.
Fig. 5 is a diagram showing spectrum measurement data and a Raman spectrum.
Fig. 6 is a block diagram of a CPU of the computer constituting the selection device.
Fig. 7 is a diagram showing processing of specifying first spectrum measurement data and second spectrum measurement data from a spectrum measurement data group based on chromatogram data.
Fig. 8 is a diagram showing the first spectrum measurement data.
Fig. 9 is a diagram showing the second spectrum measurement data.
Fig. 10 is a diagram showing processing of calculating difference data between the first spectrum measurement data and the second spectrum measurement data.
Fig. 11 is a diagram showing processing of comparing the difference data with a threshold value and selecting a specific wave number band of an aggregate.
Fig. 12 is a diagram showing processing of comparing the difference data with the threshold value and selecting the specific wave number band of the aggregate on the Raman spectrum.
Fig. 13 is a block diagram of a CPU of the computer constituting the learning device.
Fig. 14 is a diagram showing a neural network constituting a concentration prediction model.
Fig. 15 is a diagram showing a composition of a data set group.
Fig. 16 is a diagram showing processing in a training phase of the concentration prediction model.
Fig. 17 is a diagram showing processing in a validation phase of the concentration prediction model.
Fig. 18 is a block diagram of a CPU of the computer constituting the operation device.
Fig. 19 is a diagram showing a composition of third spectrum measurement data.
Fig. 20 is a diagram showing processing of generating input data from the third spectrum measurement data with reference to specific wave number band data, inputting the input data to the concentration prediction model, and outputting a concentration prediction result from the concentration prediction model.
Fig. 21 is a diagram showing a Raman spectrum analysis screen.
Fig. 22 is a diagram showing the Raman spectrum analysis screen on which the concentration prediction result is displayed.
Fig. 23 is a flowchart showing a processing procedure of the selection device.
Fig. 24 is a flowchart showing a processing procedure of the learning device.
Fig. 25 is a flowchart showing a processing procedure of the operation device.
Fig. 26 is a diagram showing another example of the composition of the third spectrum measurement data.
Fig. 27 is a table showing an outline of Example and Comparative Example.

### DESCRIPTION OF THE PREFERRED EMBODIMENTS

### [First Embodiment]

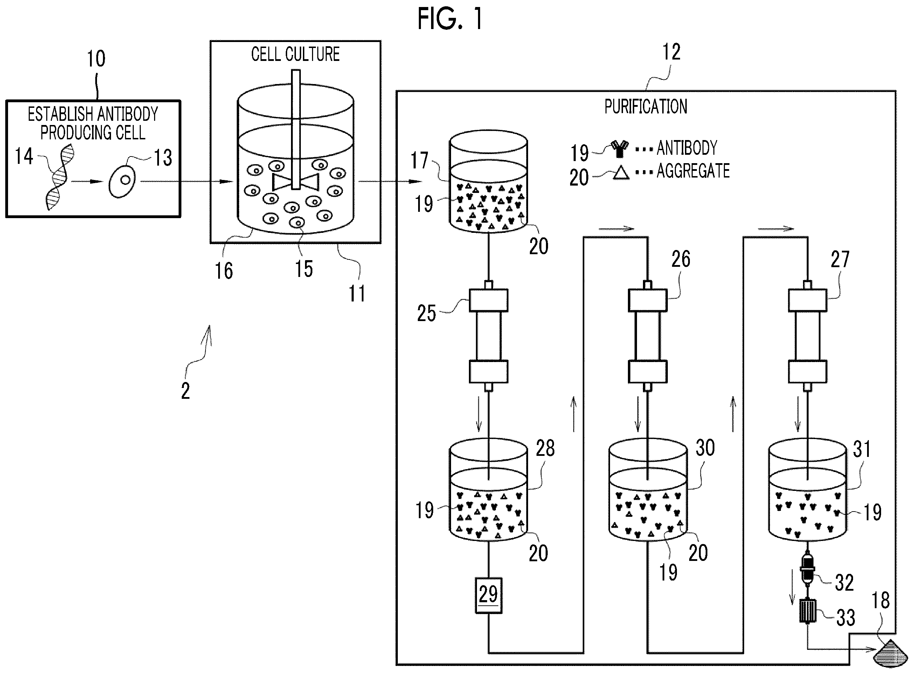

As shown in Fig. 1 as an example, a manufacturing process 2 of a biopharmaceutical is roughly divided into a first process 10, a second process 11, and a third process 12. The first process 10 is a process of incorporating an antibody gene 14 into a cell 13 such as Chinese hamster ovary (CHO) cells to establish an antibody producing cell 15. The second process is a process of cell culture of the antibody producing cell 15 in a culture tank 16.

The third process 12 is a process of purifying a drug substance 18 of the biopharmaceutical from a culture supernatant solution 17. The culture supernatant solution 17 is a solution obtained by removing cells from a culture solution in the culture tank 16 after the second process 11 ends. The immunoglobulins produced by the antibody producing cell 15, that is, antibodies 19 are dispersed in the culture supernatant solution 17. The antibody 19 is, for example, a monoclonal antibody, and serves as an active ingredient of the biopharmaceutical. In addition, an aggregate 20 of the antibody 19 are also dispersed in the culture supernatant solution 17. The antibody 19 is an example of a "target protein" according to the present disclosed technology. The aggregate 20 is an example of a "target component" according to the present disclosed technology.

The aggregate 20 is an aggregate of the antibody 19 itself and/or aggregation of a plurality of denatured forms of the antibody 19 with an amino acid sequence that is 70% or more identical to that of the antibody 19. Therefore, the aggregate 20 has a greater mass than the antibody 19. The aggregate 20 has a greater molecular weight than the antibody 19. Specifically, the aggregate 20 is a substance of which a molecular weight is 1.2 times or more the molecular weight of the antibody 19. Further, the aggregate 20 is a substance having a molecular weight of preferably 1.5 times or more, more preferably 1.8 times or more, and particularly preferably 1.9 times or more the molecular weight of the antibody 19. It should be noted that, although not shown, in the culture supernatant solution 17, in addition to the antibody 19 and the aggregate 20, a cell-derived protein, a cell-derived deoxyribonucleic acid (DNA), a virus, and the like are also dispersed.

In the third process 12, the culture supernatant solution 17 is continuously or intermittently purified by an immunoaffinity chromatography device 25, a cation chromatography device 26, an anion chromatography device 27, and the like. The culture supernatant solution 17 is introduced into the immunoaffinity chromatography device 25. The immunoaffinity chromatography device 25 extracts the antibody 19 from the culture supernatant solution 17 by using a column in which a ligand such as a protein A having an affinity for the antibody 19 is immobilized on a carrier, and thereby generating a first purified solution 28. The first purified solution 28 is subjected to a virus inactivation treatment 29. The first purified solution 28 is an example of a "suspension" according to the present disclosed technology.

The first purified solution 28 after being subjected to the virus inactivation treatment 29 is introduced into the cation chromatography device 26. The cation chromatography device 26 extracts the antibody 19 from the first purified solution 28 by using a column having a cation exchanger as a stationary phase, to generate a second purified solution 30. The second purified solution 30 is an example of a "suspension" according to the present disclosed technology.

The second purified solution 30 is introduced into the anion chromatography device 27. The anion chromatography device 27 extracts the antibody 19 from the second purified solution 30 by using a column having an anion exchanger as a stationary phase, to generate a third purified solution 31.

The third purified solution 31 passes through a filter 32, so that the virus is removed. Thereafter, the third purified solution 31 is subjected to a concentration/filtration treatment via ultrafiltration (UF) and diafiltration (DF) using the filter 33. As a result, the drug substance 18 of the biopharmaceutical is obtained. By sequentially performing the component separation treatments via a plurality of types of chromatography devices 25 to 27, impurities such as the aggregate 20 and viruses are removed in stages from the culture supernatant solution 17, and the purity of the antibody 19 is increased in stages. It should be noted that a single pass tangential flow filtration (SPTFF) type filter may be provided in front of the immunoaffinity chromatography device 25.

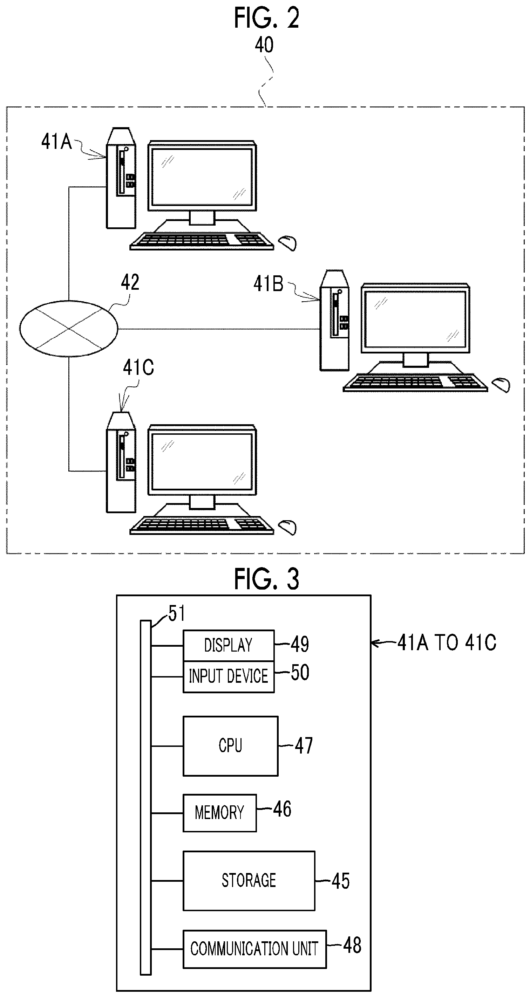

As shown in Fig. 2 as an example, the information processing system 40 is configured by a selection device 41A, a learning device 41B, and an operation device 41C. These devices are connected to each other via a network 42 in a communicable manner. The network 42 is, for example, a wide area network (WAN) such as the Internet or a public communication network. The selection device 41A, the learning device 41B, and the operation device 41C are, for example, a desktop personal computer, a laptop personal computer, or a tablet terminal.

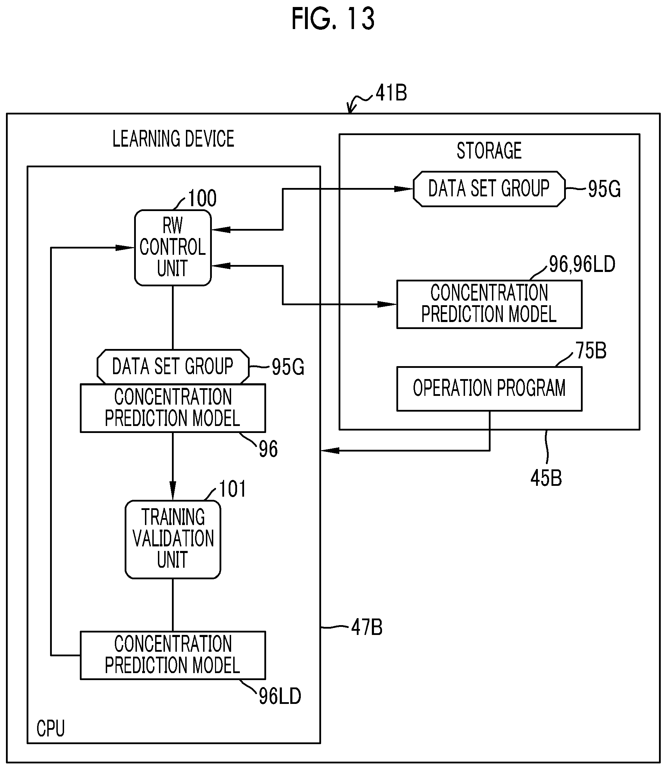

The selection device 41A implements processing of selecting a specific wave number band specific to the aggregate 20 among the wave numbers of the Raman spectrum. The learning device 41B performs processing of training a concentration prediction model 96 (see Fig. 13) that predicts the concentration of the aggregate 20. The operation device 41C implements processing of predicting the concentration of the aggregate 20 using a trained concentration prediction model 96LD (see Fig. 13). The concentration is an example of a "state" according to the present disclosed technology. The "state" is an indicator representing physicochemical features of the target component. In addition, the selection device 41A, the learning device 41B, and the operation device 41C are examples of an "information processing device" according to the present disclosed technology. As described above, the "information processing device" according to the present disclosed technology may be implemented across a plurality of devices.

As shown in Fig. 3 as an example, computers constituting the selection device 41A, the learning device 41B, and the operation device 41C basically have the same configuration, and comprise a storage 45, a memory 46, a central processing unit (CPU) 47, a communication unit 48, a display 49, and an input device 50. These units are connected to each other via a busline 51.

The storage 45 is a hard disk drive that is built in the computers constituting the selection device 41A, the learning device 41B, and the operation device 41C, or is connected to the computers through a cable or a network. Alternatively, the storage 45 is a disk array in which a plurality of hard disk drives are mounted. The storage 45 stores a control program such as an operating system, various application programs, various data associated with these programs, and the like. A solid-state drive may be used instead of the hard disk drive.

The memory 46 is a work memory for the CPU 47 to execute processing. The CPU 47 loads the program stored in the storage 45 into the memory 46, and executes processing in accordance with the program. Accordingly, the CPU 47 integrally controls the respective units of the computer. The CPU 47 is an example of a "processor" according to the present disclosed technology. It should be noted that the memory 46 may be built in the CPU 47.

The communication unit 48 is a network interface that performs control of transmitting various types of information via the network 42 and the like. The display 49 displays various screens. Various screens have an operation function via a graphical user interface (GUI). The computers constituting the selection device 41A, the learning device 41B, and the operation device 41C receive an input of an operation instruction from the input device 50 through various screens. The input device 50 is, for example, a keyboard, a mouse, a touch panel, and a microphone for voice input.

It should be noted that, in the following description, the respective units (the storage 45 and the CPU 47) of the computer constituting the selection device 41A are distinguished by a reference numeral with a subscript "A", the respective units (the storage 45 and the CPU 47) of the computer constituting the learning device 41B are distinguished by a reference numeral with a subscript "B", and the respective units (the storage 45, the CPU 47, and the display 49) of the computer constituting the operation device 41C are distinguished by a reference numeral with a subscript "C".

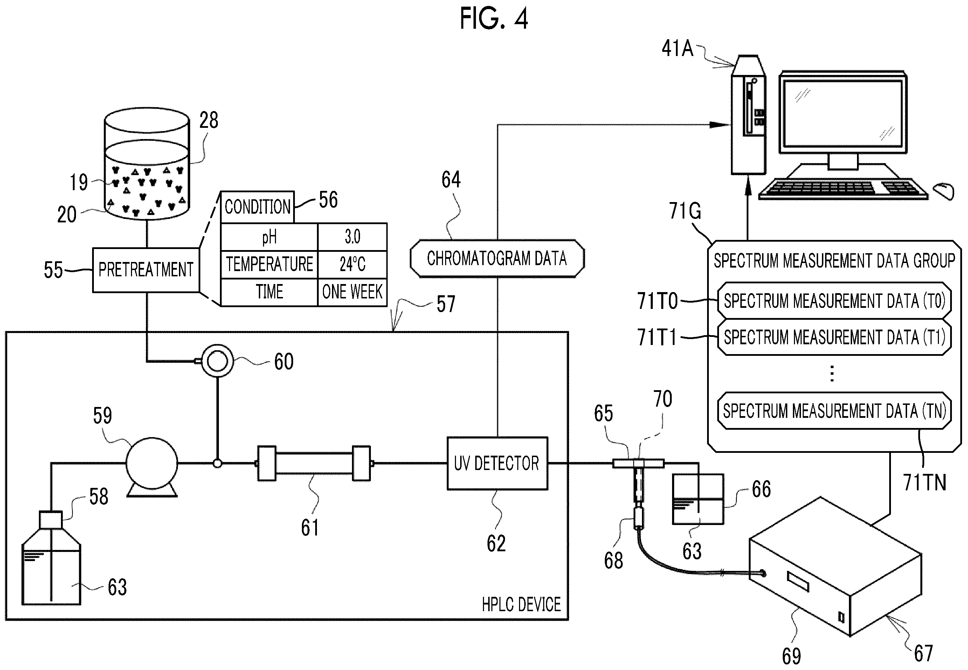

As shown in Fig. 4 as an example, the first purified solution 28 after an immunoaffinity chromatography treatment, which is output from the immunoaffinity chromatography device 25, is used for the selection of the specific wave number band of the aggregate 20. The first purified solution 28 is subjected to a pretreatment 55 for promoting the generation of the aggregate 20. Specifically, the pretreatment 55 is a treatment in which the hydrogen ion exponent (denoted by pH in Fig. 4; potential hydrogen) of the first purified solution 28 is set to 3.0, and the first purified solution 28 is allowed to stand in an environment of a temperature of 24°C for one week, as shown in Table 56. After the pretreatment 55 is performed, the first purified solution 28 is introduced into a high-performance liquid chromatography (HPLC) device 57. It should be noted that the generation of the aggregate 20 of the first purified solution 28 may be further promoted, for example, by raising the temperature to 30°C or higher.

The HPLC device 57 includes a reservoir 58, a pump 59, an autosampler 60, a column 61, and an ultraviolet detector (hereinafter, referred to as UV detector) 62. A liquid 63, which is in a mobile phase, is stored in the reservoir 58. Examples of the liquid 63 include phosphate-buffered saline (PBS). The pump 59 feeds the liquid 63 in the reservoir 58 to the column 61 at a flow rate set in advance (for example, 1 mL/min).

The autosampler 60 is connected between the pump 59 and the column 61. The autosampler 60 automatically injects a preset amount (for example, several µL to several tens of µL) of the first purified solution 28 after being subjected to the pretreatment 55, into the liquid 63 flowing toward the column 61. It should be noted that an injector that manually injects the first purified solution 28 may be used instead of the autosampler 60.

The column 61 contains a filler (for example, silica gel, a synthetic resin, or the like) as a stationary phase for separating the antibody 19 and the aggregate 20 in the first purified solution 28, and gel filtration chromatography or size exclusion chromatography can be performed. The antibody 19 and the aggregate 20 separated by the column 61 are sequentially eluted from the column 61 along with the liquid 63, to reach the UV detector 62. The UV detector 62 irradiates the liquid 63 from the column 61 with detection light, and measures the absorbance (light absorption amount) of a substance in the liquid 63. The detection light is ultraviolet light and/or visible light having a wavelength (light having a wavelength of 190 nm to 800 nm, and more specifically, light having a wavelength of 280 nm) corresponding to the antibody 19 and the aggregate 20.

The UV detector 62 is connected to the selection device 41A through a computer network, such as a local area network (LAN), in a communicable manner. The UV detector 62 transmits chromatogram data 64 that is a measurement result of the absorbance, to the selection device 41A.

A flow cell 65 is connected downstream of the UV detector 62. The liquid 63, which has passed through the UV detector 62, flows through the flow cell 65. A collection tank 66 for the liquid 63 is connected to downstream of the flow cell 65.

A probe 68 of the Raman spectrometer 67 is connected to the flow cell 65. A Raman spectrometer 67 is a device that evaluates a substance by using characteristics of Raman scattered light. In a case in which the substance is irradiated with excitation light, the Raman scattered light having a different wavelength from the excitation light is generated by an interaction between the excitation light and the substance. A wavelength difference between the excitation light and the Raman scattered light corresponds to an energy of molecular vibration possessed by the substance. Therefore, it is possible to obtain the Raman scattered light having different wave numbers between the substances having different molecular structures. Out of a Stokes ray and an anti-Stokes ray, it is preferable to use the Stokes ray as the Raman scattered light. The Raman scattered light is an example of an "electromagnetic wave" according to the present disclosed technology. In addition, the spectrum of the Raman scattered light, that is, the Raman spectrum is an example of a "spectrum" according to the present disclosed technology.

The Raman spectrometer 67 is configured by a probe 68 and an analyzer 69. The probe 68 emits the excitation light from an emission port at a distal end thereof to the liquid 63 flowing through the measurement unit 70 of the flow cell 65. Then, the Raman scattered light generated by the interaction between the excitation light and the substance in the liquid 63 is received by a light receiving portion disposed at the distal end. The probe 68 outputs the received Raman scattered light to the analyzer 69. In this example, laser light is used as the excitation light, the output of the laser light is set to 200 mW, an excitation wavelength is set to 785 nm, and an irradiation time is set to 1 second.

The analyzer 69 generates spectrum measurement data 71 by decomposing the Raman scattered light for each wave number and deriving the intensity value of the Raman scattered light for each wave number. Here, the probe 68 emits the excitation light at a preset interval and receives Raman scattered light from a time T0 when the injection of the first purified solution 28 is started by the autosampler 60 to a time TN sufficient for the UV detector 62 to measure the absorbance of the antibody 19 and the aggregate 20. The analyzer 69 generates the spectrum measurement data 71 each time. Therefore, a plurality of pieces of spectrum measurement data 71 are generated, such as spectrum measurement data 71T0 at a time T0, spectrum measurement data 71T1 at a time T1, ..., and the spectrum measurement data 71TN at a time TN.

The analyzer 69 is connected to the selection device 41A through a computer network, such as LAN, in a communicable manner, similarly to the HPLC device 57. The analyzer 69 transmits a spectrum measurement data group 71G, which is a set of the plurality of pieces of spectrum measurement data 71, to the selection device 41A.

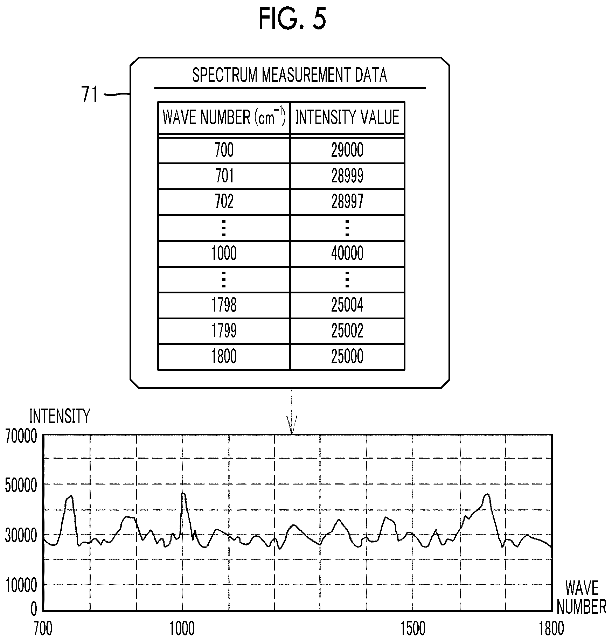

As shown in Fig. 5 as an example, the spectrum measurement data 71 is data in which the intensity value of the Raman scattered light is registered for each wave number. In Fig. 5, the spectrum measurement data 71 is data in which the intensity values of the scattered light in a range of wave numbers of 700 cm⁻¹ to 1800 cm⁻¹ are derived in increments of 1 cm⁻¹. It should be noted that the graph shown in the lower part of Fig. 5 is obtained by plotting the intensity value of the spectrum measurement data 71 for each wave number and connecting the points with a line, that is, represents the Raman spectrum.

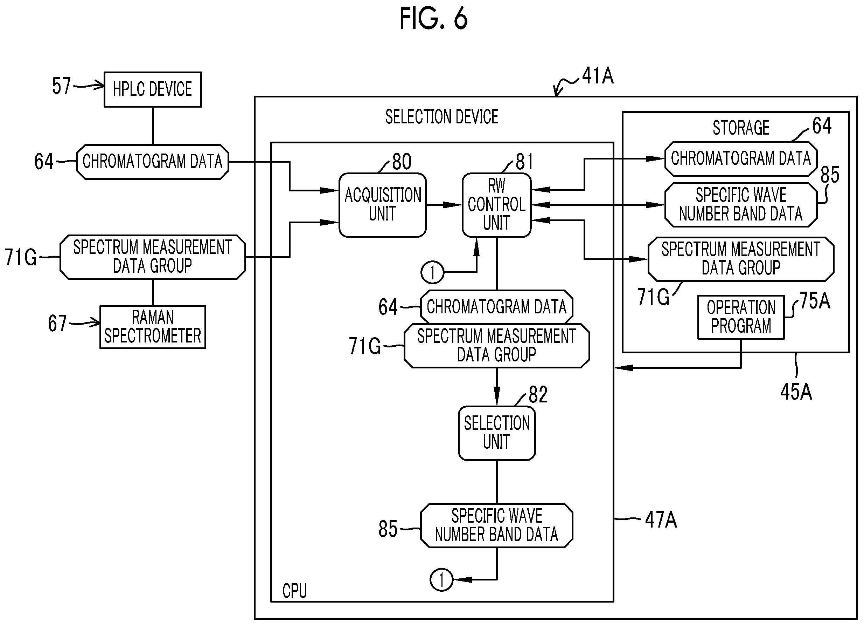

As shown in Fig. 6 as an example, a storage 45A of the selection device 41A stores an operation program 75A. The operation program 75A is an application program causing the computer to function as the selection device 41A. In other words, the operation program 75A is an example of an "operation program of an information processing device" according to the present disclosed technology.

In a case in which the operation program 75A is started, the CPU 47A of the computer constituting the selection device 41A functions as an acquisition unit 80, a read/write control unit (hereinafter, referred to as a read/write (RW) control unit) 81, and a selection unit 82 in cooperation with the memory 46 and the like.

The acquisition unit 80 acquires the chromatogram data 64 from the HPLC device 57, and the spectrum measurement data group 71G from the Raman spectrometer 67. The acquisition unit 80 outputs the chromatogram data 64 and the spectrum measurement data group 71G to the RW control unit 81.

The RW control unit 81 controls the storage of various types of data in the storage 45A and the readout of various types of data stored in the storage 45A. The RW control unit 81 stores, in the storage 45A, the chromatogram data 64 and the spectrum measurement data group 71G from the acquisition unit 80. The RW control unit 81 reads out the chromatogram data 64 and the spectrum measurement data group 71G from the storage 45A, and outputs the readout chromatogram data 64 and the readout spectrum measurement data group 71G to the selection unit 82.

The selection unit 82 selects the specific wave number band of the aggregate 20 based on the chromatogram data 64 and the spectrum measurement data group 71G. The selection unit 82 generates specific wave number band data 85 as a selection result of the specific wave number band. The selection unit 82 outputs the specific wave number band data 85 to the RW control unit 81. The RW control unit 81 stores the specific wave number band data 85 in the storage 45A.

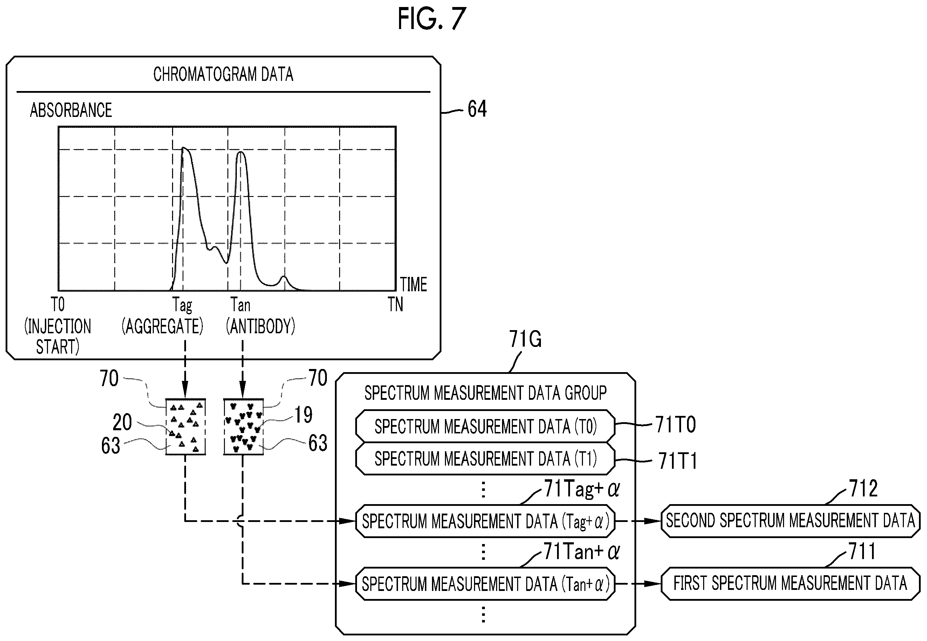

As shown in Fig. 7 as an example, the selection unit 82 specifies first spectrum measurement data 711 and second spectrum measurement data 712, from among the plurality of pieces of spectrum measurement data 71 in the spectrum measurement data group 71G, based on the chromatogram data 64. The first spectrum measurement data 711 is data obtained by measuring the Raman spectrum emitted from the antibody 19. The second spectrum measurement data 712 is data obtained by measuring the Raman spectrum emitted from the aggregate 20.

The selection unit 82 derives, from the chromatogram data 64, a time Tan (retention time of the antibody 19) at which a peak of the absorbance indicating the antibody 19 is expressed and a time Tag (retention time of the aggregate 20) at which a peak of the absorbance indicating the aggregate 20 is expressed. The selection unit 82 specifies spectrum measurement data 71Tan+α in which the Raman spectrum of the liquid 63 that has flowed through the measurement unit 70 of the flow cell 65 is measured at the time Tan, as the first spectrum measurement data 711. In addition, the selection unit 82 specifies spectrum measurement data 71Tag+α in which the Raman spectrum of the liquid 63 that has flowed through the measurement unit 70 of the flow cell 65 is measured at the time Tag, as the second spectrum measurement data 712. Here, the liquid 63 that has flowed through the measurement unit 70 of the flow cell 65 at the time Tan is an example of a "first solution" according to the present disclosed technology. In addition, the liquid 63 that has flowed through the measurement unit 70 of the flow cell 65 at the time tag is an example of a "second solution" according to the present disclosed technology. In addition, "+α" of the time Tan+α and Tag+α is a time lag from the measurement of the absorbance via the UV detector 62 to the measurement of the Raman spectrum via the Raman spectrometer 67 in the measurement unit 70 of the flow cell 65.

It should be noted that the method of generating the liquid 63 containing the antibody 19 and the liquid 63 containing the aggregate 20 is not limited to the method using the HPLC device 57. For example, the liquid 63 containing the antibody 19 and the liquid 63 containing the aggregate 20 may be separated from the first purified solution 28 using a centrifugal ultrafiltration filter.

In this way, the spectrum measurement data group 71G includes the first spectrum measurement data 711 and the second spectrum measurement data 712. Therefore, the acquisition unit 80 acquires the spectrum measurement data group 71G, and thereby acquiring the first spectrum measurement data 711 and the second spectrum measurement data 712.

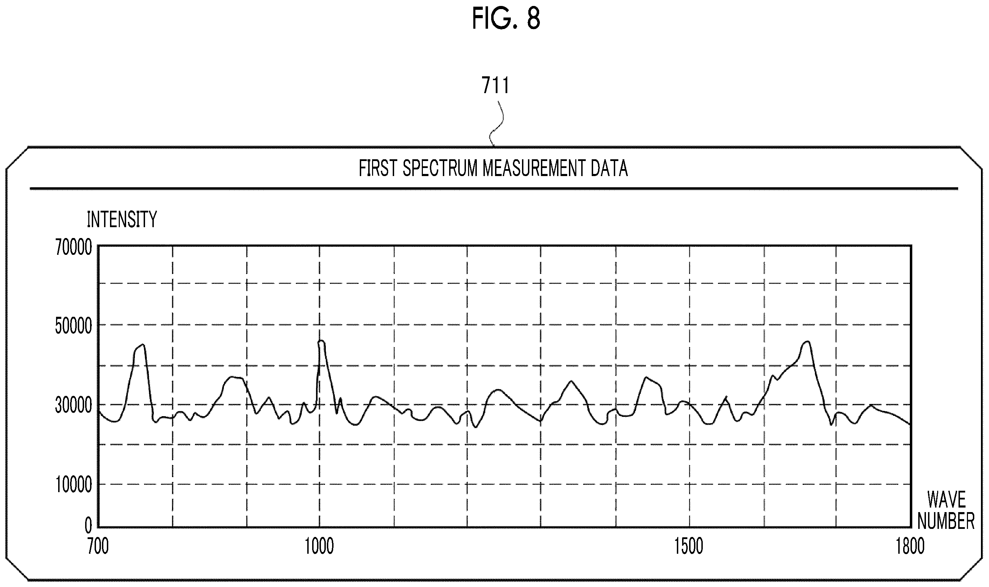

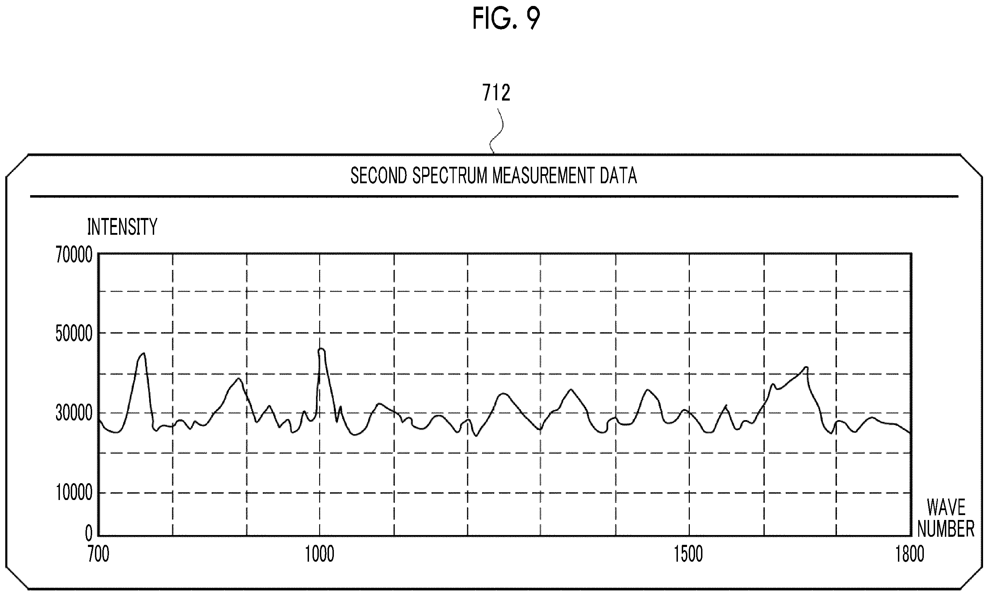

Fig. 8 shows an example of the first spectrum measurement data 711, and Fig. 9 shows an example of the second spectrum measurement data 712. As can be seen by comparing Figs. 8 and 9, the first spectrum measurement data 711 and the second spectrum measurement data 712 are substantially the same as each other, but the former is based on the antibody 19 and the latter is based on the aggregate 20, and thus the data is slightly different in some places.

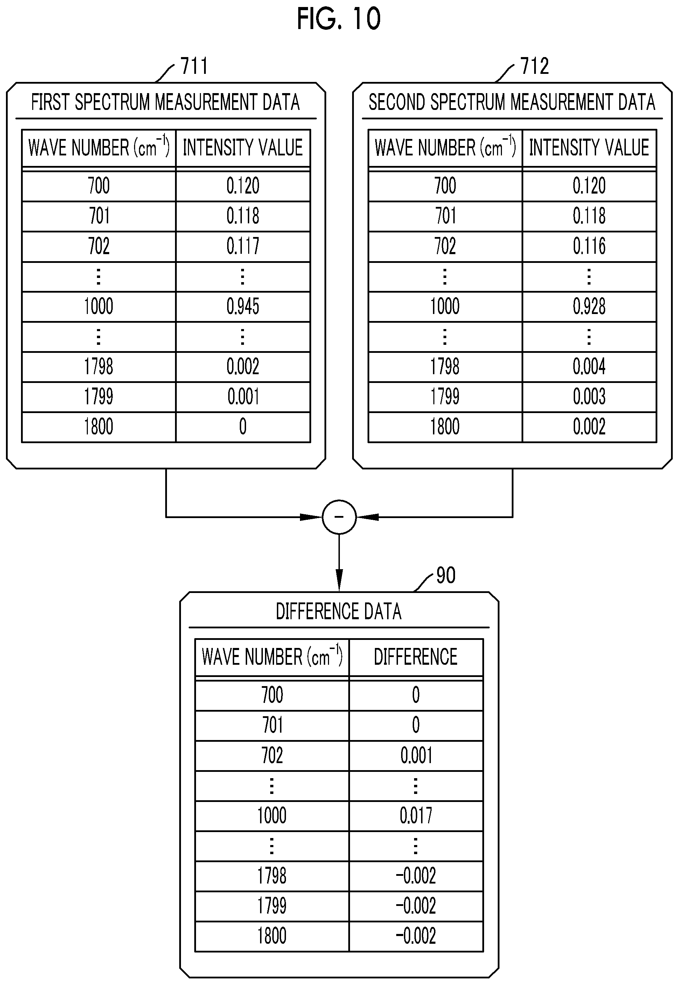

As shown in Fig. 10 as an example, the selection unit 82 calculates difference data 90 of the intensity values of each wave number of the first spectrum measurement data 711 and the second spectrum measurement data 712. The difference data 90 is data in which a difference, which is obtained by subtracting the intensity value of the second spectrum measurement data 712 from the intensity value of the first spectrum measurement data 711, is registered for each wave number. It should be noted that the selection unit 82 normalizes the first spectrum measurement data 711 and the second spectrum measurement data 712 by setting a maximum value of the intensity value to 1 and a minimum value to 0, before the calculation of the difference data 90.

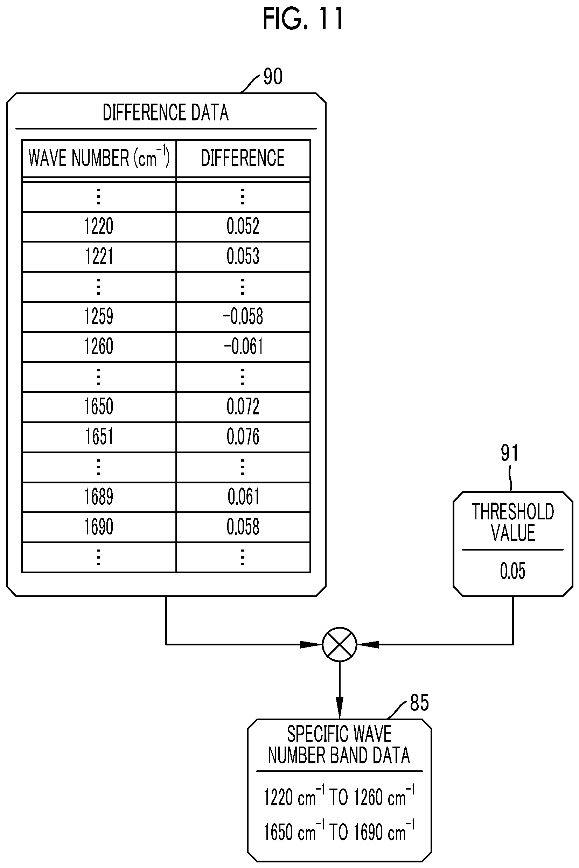

As shown in Fig. 11 as an example, the selection unit 82 compares an absolute value of the difference in the difference data 90 with a threshold value 91 set in advance. Then, the wave number band in which the absolute value of the difference is equal to or more than the threshold value is selected as the specific wave number band of the aggregate 20. Fig. 11 shows a case in which 0.05 is set as the threshold value, and 1220 cm⁻¹ to 1260 cm⁻¹ and 1650 cm⁻¹ to 1690 cm⁻¹ are selected as the specific wave number bands. It should be noted that the specific wave number band is not particularly limited as long as the specific wave number band is in a range of 700 cm⁻¹ to 1800 cm⁻¹, but is preferably in a range of 1220 cm⁻¹ to 1690 cm⁻¹, and more preferably in a range of 1220 cm⁻¹ to 1260 cm⁻¹ and a range of 1650 cm⁻¹ to 1690 cm⁻¹ as described above. In addition, the specific wave number band is preferably in two or more ranges, such as 1220 cm⁻¹ to 1260 cm⁻¹ and 1650 cm⁻¹ to 1690 cm⁻¹ as described above. A range in which a band of phenylalanine appears, a range in which a band of tryptophan appears, a range in which a band of tyrosine appears, or the like may be selected as the specific wave number band.

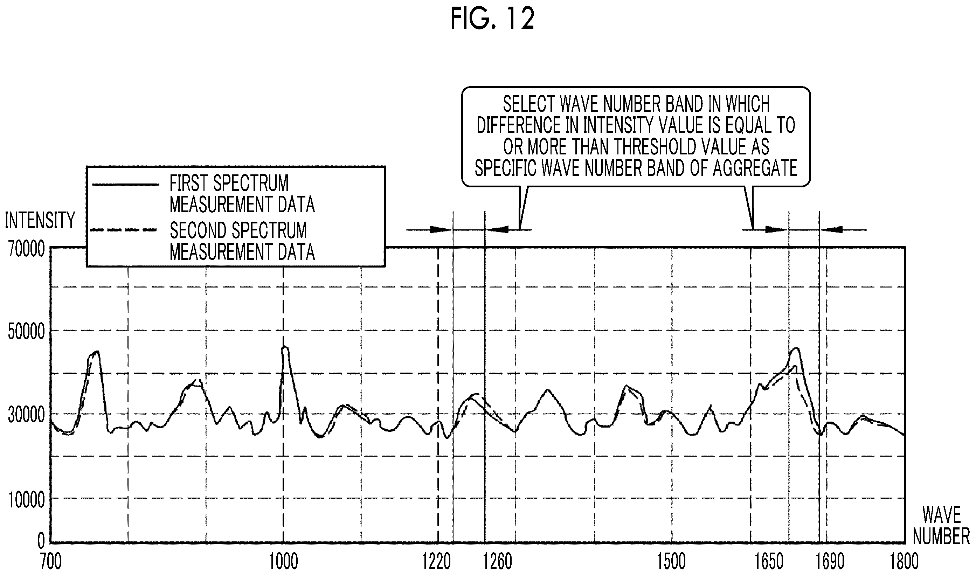

Fig. 12 is a view showing the processing shown in Fig. 11 of comparing the difference data 90 with the threshold value 91 and selecting the specific wave number band of the aggregate on the Raman spectra of the first spectrum measurement data 711 and the second spectrum measurement data 712.

It should be noted that a ratio between the intensity value of each wave number of the first spectrum measurement data 711 and the intensity value of each wave number of the second spectrum measurement data 712 may be calculated, and the wave number band in which the ratio deviates from 1 by a threshold value or more may be selected as the specific wave number band of the aggregate 20.

As shown in Fig. 13 as an example, a storage 45B of the learning device 41B stores an operation program 75B. The operation program 75B is an application program causing the computer to function as the learning device 41B. That is, the operation program 75B is an example of an "operation program of an information processing device" according to the present disclosed technology, similarly to the operation program 75A. The storage 45B stores a data set group 95G and a concentration prediction model 96, in addition to the operation program 75B. The concentration prediction model 96 is an example of a "state prediction model" according to the present disclosed technology.

In a case in which the operation program 75B is started, the CPU 47B of the computer constituting the learning device 41B functions as an RW control unit 100 and a training validation unit 101 in cooperation with the memory 46 and the like.

The RW control unit 100 controls the storage of various types of data in the storage 45B and the readout of various types of data stored in the storage 45B, similarly to the RW control unit 81 of the selection device 41A. The RW control unit 100 reads out the data set group 95G and the concentration prediction model 96 from the storage 45B, and outputs the readout data set group 95G and the readout concentration prediction model 96 to the training validation unit 101.

The training validation unit 101 performs training and validation of the concentration prediction model 96 using the data set group 95G. The training validation unit 101 outputs the trained concentration prediction model 96LD obtained by performing the training and the validation, to the RW control unit 100. The RW control unit 100 stores the concentration prediction model 96LD in the storage 45B.

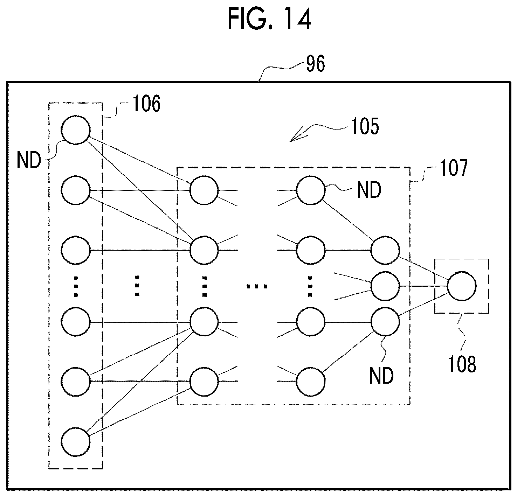

As shown in Fig. 14 as an example, the concentration prediction model 96 is constructed by a neural network 105. Therefore, the concentration prediction model 96 is also an example of a "machine learning model" according to the present disclosed technology. The neural network 105 includes, as is well known, an input layer 106, an intermediate layer (also referred to as a hidden layer) 107, and an output layer 108. Each of the input layer 106, the intermediate layer 107, and the output layer 108 includes a plurality of nodes ND. A coefficient indicating the strength of the connection of the respective nodes ND is set between the node ND of the input layer 106 and the node ND of the intermediate layer 107, between the nodes ND in the intermediate layer 107, and between the node ND of the intermediate layer 107 and the node ND of the output layer 108. A suitable activation function, such as a linear function or a rectified linear unit (ReLU) function, is set for the node ND of the output layer 108.

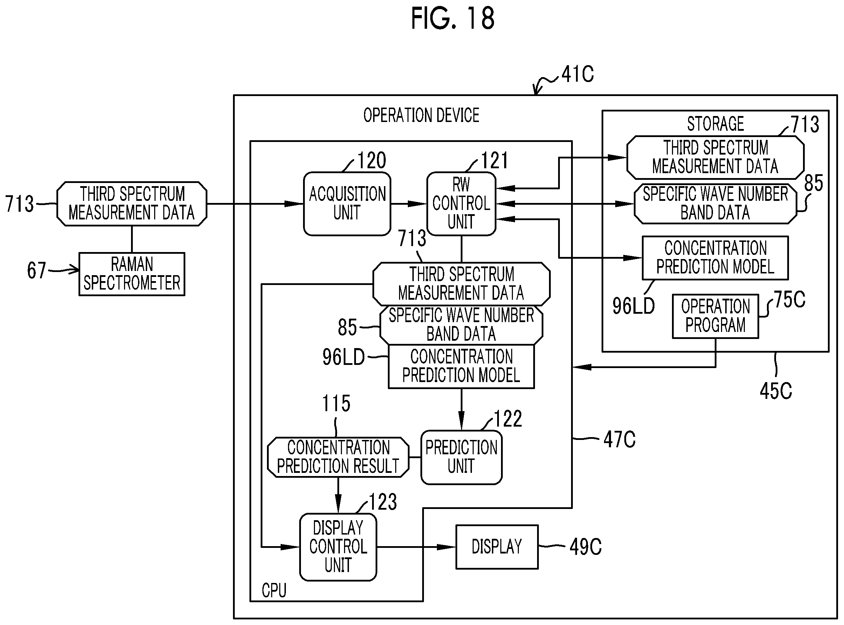

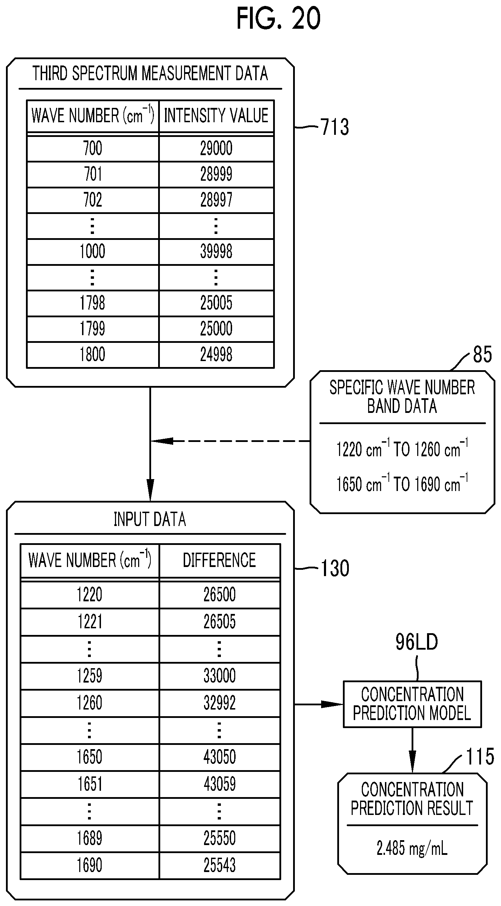

In each node ND of the input layer 106, the intensity value of the specific wave number band, among the intensity values of each wave number of the spectrum measurement data 71, is input as input data 130 (see Fig. 20). In addition, a concentration prediction result 115 (see Fig. 18), which is a prediction result of the concentration of the aggregate 20, is output from the node ND of the output layer 108.

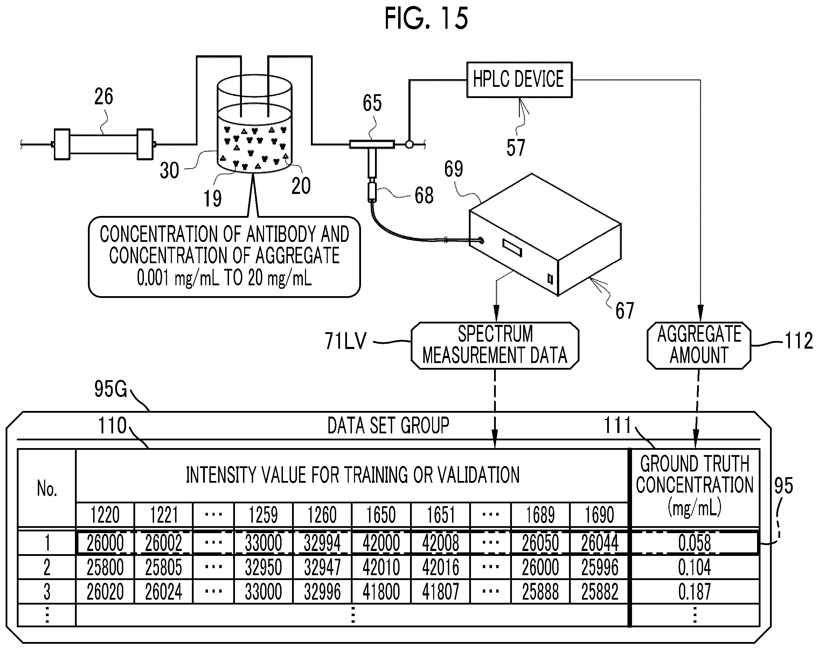

As shown in Fig. 15 as an example, the data set group 95G includes a plurality of data sets 95. The data set 95 is composed of an intensity value for training or validation 110 and a ground truth concentration 111. The intensity value for training or validation 110 is obtained by extracting the intensity value of the specific wave number band selected in the selection device 41A from the intensity value of each wave number of spectrum measurement data 71LV for generating the data set 95. The spectrum measurement data 71LV is data obtained by measuring the Raman spectrum of the second purified solution 30 after the cation chromatography treatment, which is output from the cation chromatography device 26, by using the flow cell 65 and the Raman spectrometer 67.

The spectrum measurement data 71LV is intermittently measured a plurality of times from a start point in time to an end point in time of the cation chromatography treatment via the cation chromatography device 26. In addition, the spectrum measurement data 71LV is measured a plurality of times by randomly changing the culture conditions of the antibody producing cell 15, the gradient width, the linear flow rate, the load amount, and the like of the cation chromatography device 26. As a result, it is possible to obtain the spectrum measurement data 71LV of a plurality of second purified solutions 30 having different concentration ratios of the antibody 19 and the aggregate 20, and thus it is possible to obtain a plurality of intensity values for training or validation 110. It should be noted that, instead of the shown method of measuring the spectrum measurement data 71LV in the flow channel using the flow cell 65, a method of fractionating the second purified solution 30 that has flowed out to an outlet of the flow channel by using a fraction collector and measuring the spectrum measurement data 71LV of the fractionated second purified solution 30 may be adopted.

Both of the concentrations of the antibody 19 and the aggregate 20 in the second purified solution 30 for measuring the spectrum measurement data 71LV are in a range of 0.001 mg/mL to 20 mg/mL. Both of the concentrations of the antibody 19 and the aggregate 20 in the second purified solution 30 need only be in a range of 0.001 mg/mL to 10000 mg/mL, preferably in a range of 0.001 mg/mL to 100 mg/mL, more preferably in a range of 0.001 mg/mL to 20 mg/mL.

The ground truth concentration 111 is a concentration calculated based on an aggregate amount 112 in the second purified solution 30 in which the spectrum measurement data 71LV is measured. The aggregate amount 112 is literally an amount of the aggregate 20, and is derived by a mass spectrometry function provided in the HPLC device 57. The ground truth concentration 111 is an example of "ground truth data" according to the present disclosed technology.

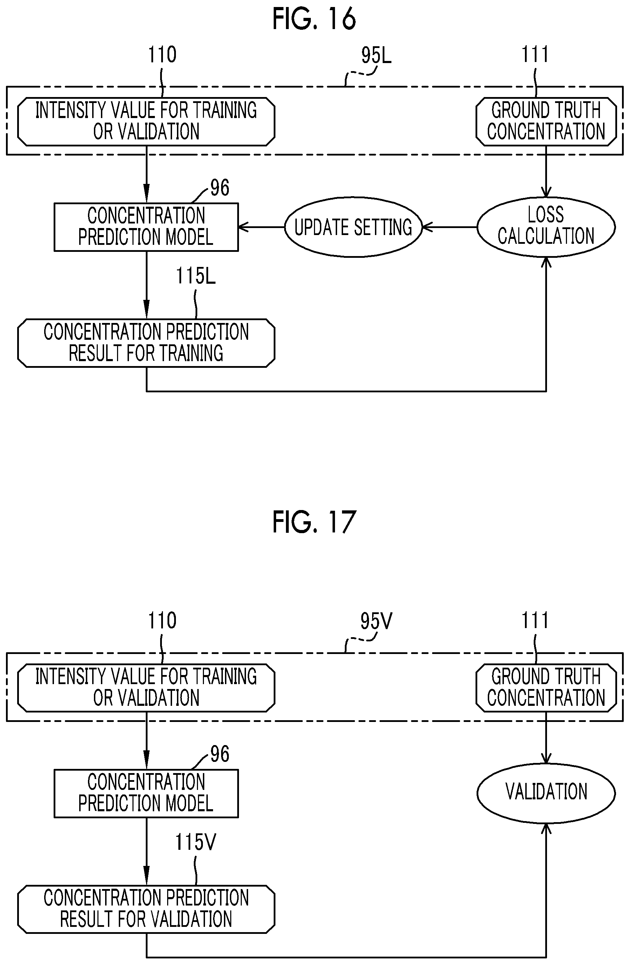

The training validation unit 101 performs cross-validation on the concentration prediction model 96 by using the plurality of data sets 95. That is, the training validation unit 101 uses m data sets of M data sets 95 as a data set for training 95L (see Fig. 16) and uses the remaining M-m data sets as a data set for validation 95V (see Fig. 17). Then, as shown in Fig. 16 as an example, the data set for training 95L is applied to the concentration prediction model 96, to train the concentration prediction model 96. In addition, as shown in Fig. 17 as an example, the data set for validation 95V is applied to the concentration prediction model 96 after being trained by applying the data set for training 95L, and the prediction accuracy of the concentration of the aggregate 20 via the concentration prediction model 96 is verified. The training validation unit 101 performs the cross-validation a set number of times while changing the configuration of the data set for training 95L and the data set for validation 95V. It is noted that m ≥ M - m and M - m = 1 may be satisfied.

As shown in Fig. 16, in the training validation unit 101, in the training phase, the intensity value for training or validation 110 in the data set for training 95L is input to the concentration prediction model 96, and the concentration prediction model 96 outputs a concentration prediction result for training 115L. The training validation unit 101 performs a loss calculation of the concentration prediction model 96 using a loss function based on a comparison result between the ground truth concentration 111 and the concentration prediction result for training 115L. The training validation unit 101 performs update setting of the coefficient between the nodes ND of the concentration prediction model 96 in accordance with the result of the loss calculation, and updates the concentration prediction model 96 in accordance with the update setting.

The training validation unit 101 repeatedly performs the series of processing of inputting the intensity value for training or validation 110 to the concentration prediction model 96, outputting the concentration prediction result for training 115L from the concentration prediction model 96, performing the loss calculation, performing the update setting, and updating the concentration prediction model 96 while changing the data set for training 95L. The training validation unit 101 performs the repetition of the series of processing m times for the number of data sets for training 95L.

As shown in Fig. 17, in a validation phase, the training validation unit 101 inputs the intensity value for training or validation 110 in the data set for validation 95V to the concentration prediction model 96, and outputs a concentration prediction result for validation 115V from the concentration prediction model 96. The training validation unit 101 verifies the prediction accuracy of the concentration of the aggregate 20 via the concentration prediction model 96 based on a comparison result between the ground truth concentration 111 and the concentration prediction result for validation 115V.

The training validation unit 101 repeatedly performs the input of the intensity value for training or validation 110 to the concentration prediction model 96, the output of the concentration prediction result for validation 115V from the concentration prediction model 96, and the validation of the prediction accuracy while changing the data set for validation 95V. The training validation unit 101 repeats the series of processing M - m times for the number of data sets for validation 95V.

The training validation unit 101 outputs the concentration prediction model 96, which has been subjected to the cross-validation a set number of times, as the concentration prediction model 96LD to the RW control unit 100. The RW control unit 100 stores the concentration prediction model 96LD in the storage 45B.

As shown in Fig. 18 as an example, a storage 45C of the operation device 41C stores an operation program 75C. The operation program 75C is an application program causing the computer to function as the operation device 41C. That is, the operation program 75C is an example of an "operation program of an information processing device" according to the present disclosed technology, similarly to the operation programs 75A and 75B. The storage 45C stores the specific wave number band data 85 from the selection device 41A and the concentration prediction model 96LD from the learning device 41B, in addition to the operation program 75C.

In a case in which the operation program 75C is started, the CPU 47C of the computer constituting the operation device 41C functions as an acquisition unit 120, an RW control unit 121, a prediction unit 122, and a display control unit 123 in cooperation with the memory 46 and the like.

The acquisition unit 120 acquires third spectrum measurement data 713 from the Raman spectrometer 67. The acquisition unit 120 outputs the third spectrum measurement data 713 to the RW control unit 121.

The RW control unit 121 controls the storage of various types of data in the storage 45C and the readout of various types of data stored in the storage 45C, similarly to the RW control unit 81 of the selection device 41A and the RW control unit 100 of the learning device 41B. The RW control unit 121 stores the third spectrum measurement data 713 from the acquisition unit 120 in the storage 45C. In addition, the RW control unit 121 reads out the specific wave number band data 85, the concentration prediction model 96LD, and the third spectrum measurement data 713 from the storage 45C, and outputs the readout specific wave number band data 85, the readout concentration prediction model 96LD, and the readout third spectrum measurement data 713 to the prediction unit 122. The RW control unit 121 outputs the third spectrum measurement data 713 to the display control unit 123.

The prediction unit 122 applies the third spectrum measurement data 713 to the concentration prediction model 96LD, to output the concentration prediction result 115 from the concentration prediction model 96LD. The prediction unit 122 outputs the concentration prediction result 115 to the display control unit 123. The concentration prediction result 115 is an example of a "prediction result" according to the present disclosed technology.

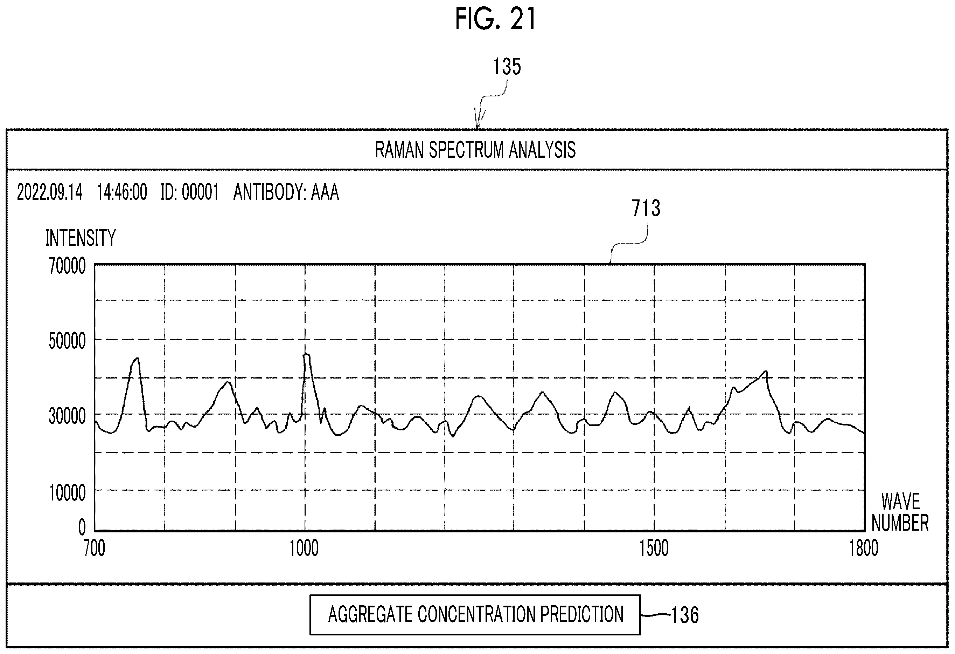

The display control unit 123 controls display of various screens on the display 49C. For example, the display control unit 123 performs control of displaying a Raman spectrum analysis screen 135 (see Fig. 21 and the like) on the display 49C.

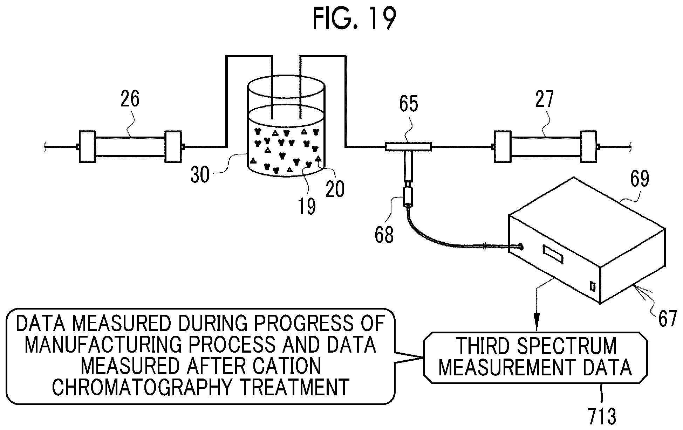

As shown in Fig. 19 as an example, the third spectrum measurement data 713 is data obtained by measuring the Raman spectrum of the second purified solution 30 of which the concentration of the aggregate 20 is unknown, by using the flow cell 65 and the Raman spectrometer 67. The flow cell 65 is installed between the cation chromatography device 26 and the anion chromatography device 27. Therefore, the second purified solution 30 is, more specifically, a liquid after the cation chromatography treatment, which is output from the cation chromatography device 26 during the progress of the manufacturing process 2. That is, the third spectrum measurement data 713 is data measured during the progress of the manufacturing process 2. In other words, the third spectrum measurement data 713 is data subjected to in-line sensing. In addition, the third spectrum measurement data 713 is data measured after the cation chromatography treatment.

As shown in Fig. 20 as an example, the prediction unit 122 generates the input data 130 by extracting the intensity value of the specific wave number band from the intensity value of each wave number of the third spectrum measurement data 713, with reference to the specific wave number band data 85. The prediction unit 122 inputs the input data 130 to the concentration prediction model 96LD, and outputs the concentration prediction result 115 from the concentration prediction model 96LD. Fig. 20 shows a case in which the specific wave number band is in a range of 1220 cm⁻¹ to 1260 cm⁻¹ and 1650 cm⁻¹ to 1690 cm⁻¹ shown in Fig. 11, and 2.485 mg/mL is output as the concentration prediction result 115.

The display control unit 123 displays, for example, the Raman spectrum analysis screen 135 shown in Fig. 21 on the display 49C in response to an instruction from a user of the operation device 41C. The third spectrum measurement data 713 is displayed on the Raman spectrum analysis screen 135.

An aggregate concentration prediction button 136 is provided at the lower part of the Raman spectrum analysis screen 135. In a case in which the aggregate concentration prediction button 136 is pressed, the CPU 47C of the operation device 41C receives an aggregate concentration prediction instruction. The CPU 47C receives the aggregate concentration prediction instruction, causes the prediction unit 122 to perform the processing shown in Fig. 20, and causes the concentration prediction model 96LD to output the concentration prediction result 115.

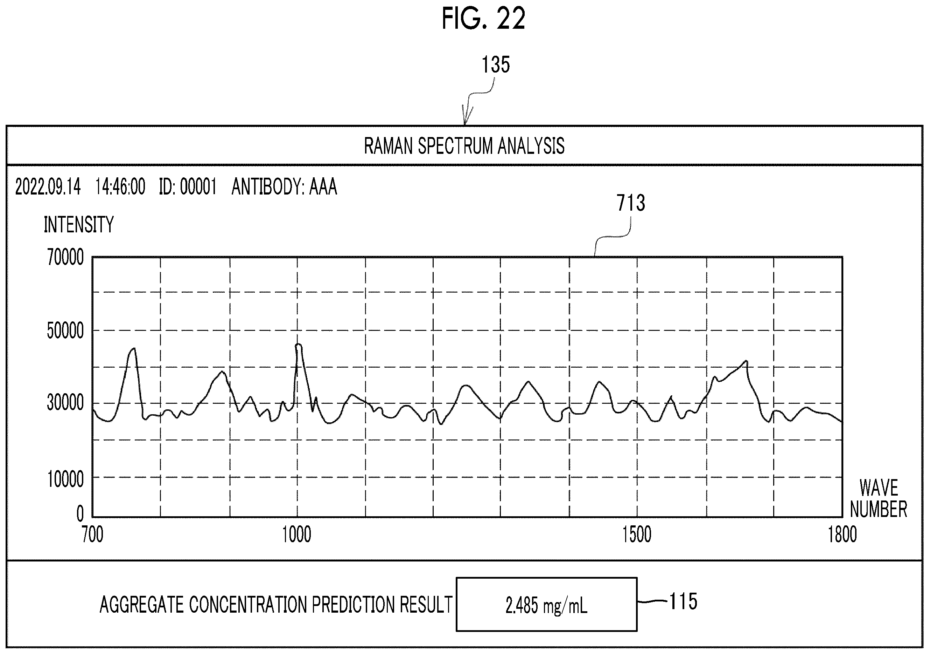

In a case in which the concentration prediction result 115 from the prediction unit 122 is input, the display control unit 123 transitions the display of the Raman spectrum analysis screen 135 as shown in Fig. 22 as an example. In Fig. 22, the concentration prediction result 115 is displayed on the Raman spectrum analysis screen 135 along with the third spectrum measurement data 713.

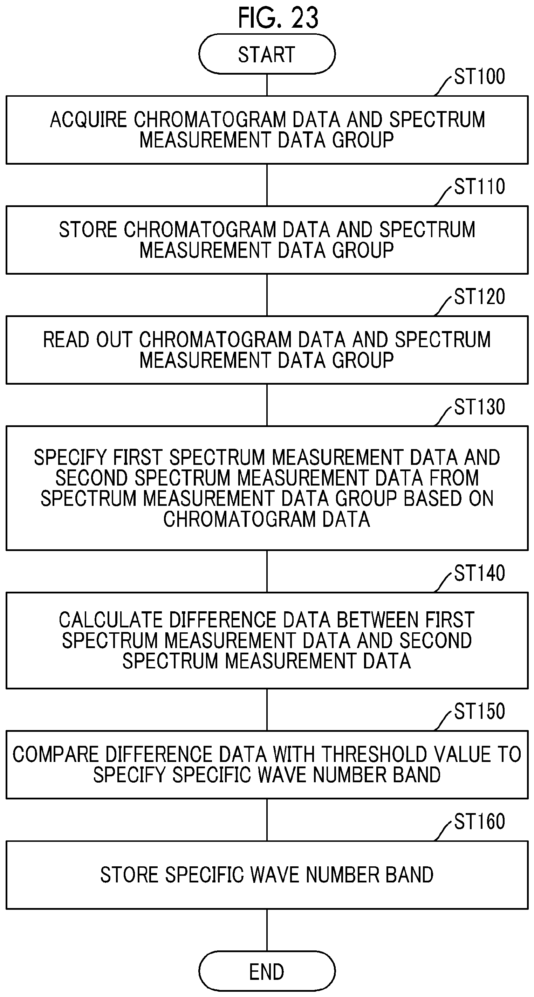

Next, an operation of the configuration described above will be described with reference to the flowchart shown in Figs. 23 to 25 as an example.

As shown in Fig. 6, the CPU 47A of the selection device 41Afunctions as the acquisition unit 80, the RW control unit 81, and the selection unit 82 via the start of the operation program 75A.

As shown in Fig. 23 as an example, in the selection device 41A, the acquisition unit 80 acquires the chromatogram data 64 from the HPLC device 57 and the spectrum measurement data group 71G from the Raman spectrometer 67, which are measured by the method shown in Fig. 4 (step ST100). The chromatogram data 64 and the spectrum measurement data group 71G are stored in the storage 45A by the RW control unit 81 (step ST110).

The chromatogram data 64 and the spectrum measurement data group 71G are read out from the storage 45A by the RW control unit 81 (step ST120), and then output to the selection unit 82. In the selection unit 82, first, as shown in Fig. 7, the first spectrum measurement data 711 and the second spectrum measurement data 712 are specified from the spectrum measurement data group 71G, based on the chromatogram data 64 (step ST130). Then, as shown in Fig. 10, the difference data 90 between the first spectrum measurement data 711 and the second spectrum measurement data 712 is calculated (step ST140). Finally, as shown in Fig. 11, the difference data 90 and the threshold value 91 are compared with each other, to select the specific wave number band of the aggregate 20 (step ST150). The specific wave number band data 85, which is the selection result of the specific wave number band, is output from the selection unit 82 to the RW control unit 81, and then stored in the storage 45A by the RW control unit 81 (step ST160).

As shown in Fig. 13, the CPU 47B of the learning device 41B functions as the RW control unit 100 and the training validation unit 101 via the start of the operation program 75B.

The data set group 95G, which is a set of the data sets 95 generated by the method shown in Fig. 15, and the concentration prediction model 96 are stored in the storage 45B of the learning device 41B. The data set group 95G and the concentration prediction model 96 are read out from the storage 45B by the RW control unit 100, and then output to the training validation unit 101.

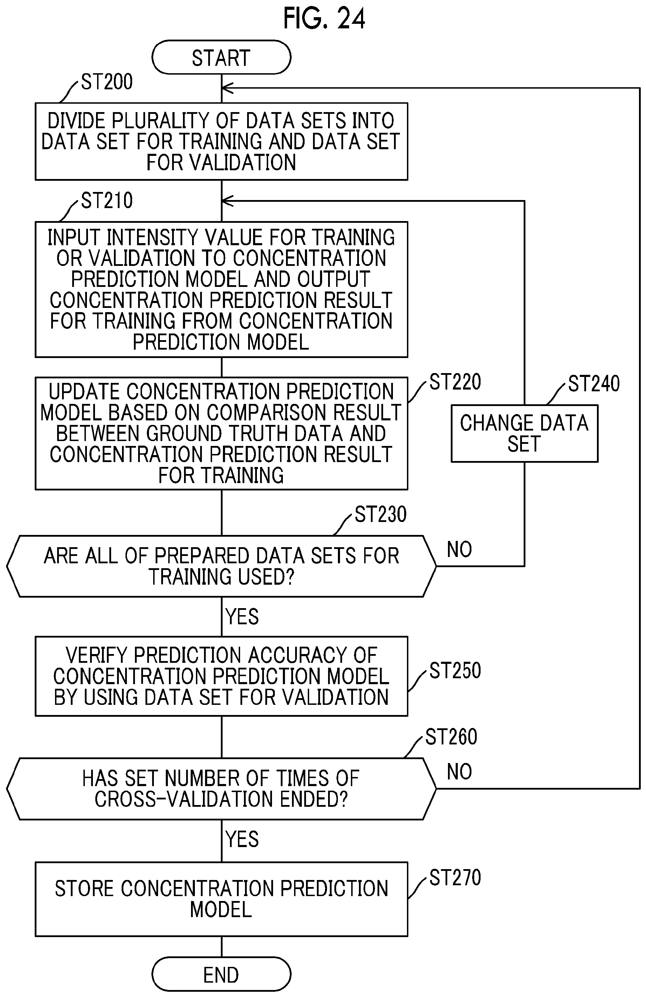

As shown in Fig. 24 as an example, in the training validation unit 101, the plurality of data sets 95 constituting the data set group 95G are divided into m data sets for training 95L and M-m data sets for validation 95V (step ST200). Then, first, the training of the concentration prediction model 96 using the data set for training 95L is performed. Specifically, as shown in Fig. 16, the intensity value for training or validation 110 of the data set for training 95L is input to the concentration prediction model 96, so that the concentration prediction result for training 115L is output from the concentration prediction model 96 (step ST210). Then, the concentration prediction model 96 is updated based on the comparison result between the ground truth concentration 111 of the data set for training 95L and the concentration prediction result for training 115L (step ST220). The processing of step ST210 and step ST220 is repeated, during a period in which the prepared data set for training 95L is not completely used (NO in step ST230), while changing the data set for training 95L (step ST240).

In a case in which all of the prepared data sets for training 95L are used (YES in step ST230), the processing proceeds to the validation of the prediction accuracy of the concentration prediction model 96 using the data set for validation 95V. Specifically, as shown in Fig. 17, the intensity value for training or validation 110 of the data set for validation 95V is input to the concentration prediction model 96, so that the concentration prediction result for validation 115V is output from the concentration prediction model 96. Then, the prediction accuracy of the concentration prediction model 96 is verified based on the comparison result between the ground truth concentration 111 of the data set for validation 95V and the concentration prediction result for validation 115V (step ST250). Although not shown, in this validation, as in a case of the training, the series of processing is repeatedly performed while the data set for validation 95V is changed until all of the prepared data sets for validation 95V are used.

The processing of step ST200 to step ST250 is repeated until the set number of times of the cross-validation ends (NO in step ST260). In a case in which the set number of times of the cross-validation ends (YES in step ST260), the concentration prediction model 96 is output from the training validation unit 101 to the RW control unit 100 as the trained concentration prediction model 96LD. The RW control unit 100 stores the concentration prediction model 96LD in the storage 45B (step ST270).

As shown in Fig. 18, the CPU 47C of the operation device 41C functions as the acquisition unit 120, the RW control unit 121, the prediction unit 122, and the display control unit 123 via the start of the operation program 75C.

The storage 45C of the operation device 41C stores the specific wave number band data 85 from the selection device 41A and the concentration prediction model 96LD from the learning device 41B. The specific wave number band data 85 and the concentration prediction model 96LD are read out from the storage 45C by the RW control unit 121, and then output to the prediction unit 122.

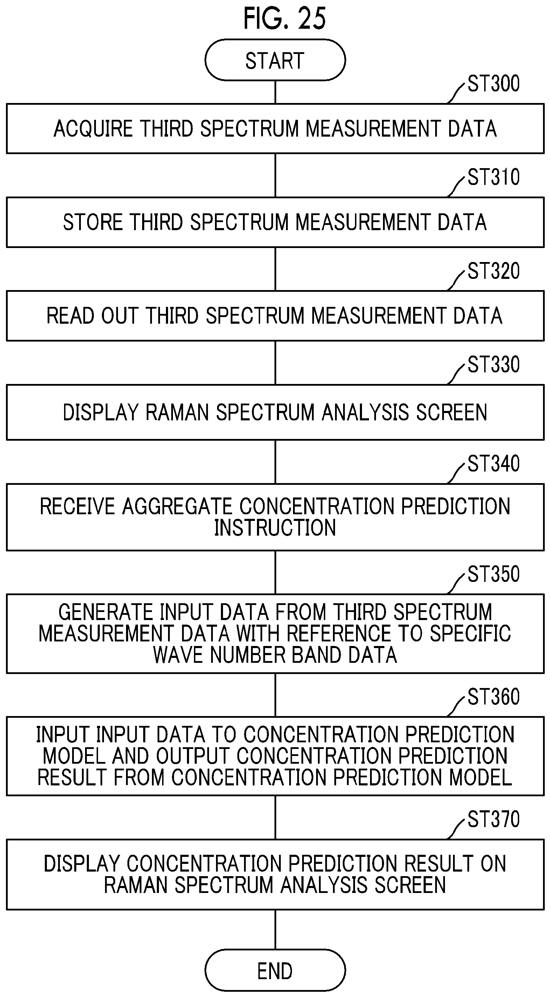

As shown in Fig. 25 as an example, in the operation device 41C, the third spectrum measurement data 713 from the Raman spectrometer 67 measured by the method shown in Fig. 19 is acquired by the acquisition unit 120 (step ST300). The RW control unit 121 stores the third spectrum measurement data 713 in the storage 45C (step ST310).

The third spectrum measurement data 713 is read out from the storage 45C by the RW control unit 121 (step ST320), and then output to the prediction unit 122 and the display control unit 123. As shown in Fig. 21, the display control unit 123 displays the Raman spectrum analysis screen 135 on the display 49C (step ST330).

The user of the operation device 41C presses the aggregate concentration prediction button 136 in order to cause the concentration prediction model 96LD to predict the concentration of the aggregate 20 in the second purified solution 30 in which the third spectrum measurement data 713 of the Raman spectrum analysis screen 135 is measured. As a result, the aggregate concentration prediction instruction is received by the CPU 47C (step ST340).

In response to the aggregate concentration prediction instruction, the prediction unit 122 generates the input data 130 from the third spectrum measurement data 713 with reference to the specific wave number band data 85, as shown in Fig. 20 (step ST350). Then, the input data 130 is input to the concentration prediction model 96LD, so that the concentration prediction result 115 is output from the concentration prediction model 96LD (step ST360). The concentration prediction result 115 is output from the prediction unit 122 to the display control unit 123, and is displayed on the Raman spectrum analysis screen 135 by the display control unit 123, as shown in Fig. 22 (step ST370).

The user makes various determinations with reference to the concentration prediction result 115 of the Raman spectrum analysis screen 135. For example, a case will be considered in which a condition setting experiment is carried out for the culture conditions of the antibody producing cell 15 by a small-scale facility and/or the purification conditions of the culture supernatant solution 17. In this case, in a case in which the concentration prediction result 115 is worse than a target value, the user makes a determination to stop the current experiment and proceed to an experiment under new conditions. In addition, a case will be considered in which the condition setting experiment is completed and mass production is performed by large-scale equipment. In this case, in a case in which the concentration prediction result 115 is worse than a target value, the user makes a determination to stop the mass production and perform the maintenance of the chromatography devices 25 to 27.

As described above, the CPU 47A of the selection device 41A comprises the acquisition unit 80 and the selection unit 82. The acquisition unit 80 and the selection unit 82 perform preparatory processing for generating the concentration prediction model 96LD that predicts the concentration of the aggregate 20 in the second purified solution 30 produced in the manufacturing process 2 of the biopharmaceutical containing the antibody 19 as the active ingredient. That is, the acquisition unit 80 acquires the first spectrum measurement data 711 in which the Raman spectrum emitted from the antibody 19 is measured, and the second spectrum measurement data 712 in which the Raman spectrum emitted from the aggregate 20 is measured. The selection unit 82 selects the specific wave number band specific to the aggregate 20 by comparing the intensity value of the first spectrum measurement data 711 with the intensity value of the second spectrum measurement data 712. Therefore, it is possible to select a rational wave number band of the spectrum measurement data 71 that is considered to contribute to the prediction of the concentration of the aggregate 20 in the second purified solution 30 produced in the manufacturing process 2 of the biopharmaceutical.

As shown in Figs. 15 to 17, the concentration prediction model 96LD is generated by using the data set 95 including the intensity value for training or validation 110, which is the intensity value of the specific wave number band, and the ground truth concentration 111 of the aggregate 20. Therefore, the concentration prediction model 96LD can be a model that outputs the concentration prediction result 115 of the aggregate 20 in accordance with the intensity value of the specific wave number band. With the concentration prediction model 96LD, it is possible to predict the concentration of the aggregate 20 in the second purified solution 30 produced in the manufacturing process 2 of the biopharmaceutical with higher accuracy than in the related art.

The concentration is the most popular indicator for knowing the physicochemical characteristics of the target component (aggregate 20). Therefore, in a case in which the concentration is predicted as the state of the target component, the user can easily understand the physicochemical characteristics of the target component.

In addition, as shown in Fig. 15, the concentrations of the antibody 19 and the aggregate 20 in the second purified solution 30 as the source of the data set 95 are both in a range of 0.001 mg/mL to 20 mg/mL. Therefore, the concentration prediction model 96LD can be set as a model that can predict a relatively low concentration with high accuracy.

As shown in Fig. 4, the first purified solution 28 to be used to select the specific wave number band is subjected to the pretreatment 55 for promoting the generation of the aggregate 20. Therefore, it is possible to reliably acquire the second spectrum measurement data 712. Since the peak of the absorbance indicating the aggregate 20 is clearly expressed in the chromatogram data 64, the second spectrum measurement data 712 can be easily specified.

As shown in Fig. 20, the concentration prediction model 96LD outputs the concentration prediction result 115 of the aggregate 20 in accordance with the intensity value of the specific wave number band of the third spectrum measurement data 713 obtained by measuring the Raman spectrum emitted from the second purified solution 30 in which the concentration of the aggregate 20 is unknown. Therefore, it is possible for the user to easily know the concentration prediction result 115 of the aggregate 20.

As shown in Fig. 19, the third spectrum measurement data 713 is data measured during the progress of the manufacturing process 2. Therefore, it is possible to save time and effort for separating the second purified solution 30 and placing the second purified solution 30 on the Raman spectrometer 67 prepared at a different place from a purification line. Further, the third spectrum measurement data 713 can be acquired without hindering the progress of the manufacturing process 2.

As shown in Fig. 19, the third spectrum measurement data 713 is data measured after the cation chromatography treatment. In the second purified solution 30 after the cation chromatography treatment, the aggregate 20 is mostly removed. Therefore, in a case in which the concentration prediction result 115 of the aggregate 20 in the second purified solution 30 after the cation chromatography treatment is high, it can be concluded that the set condition of the condition setting experiment is inappropriate or the cation chromatography device 26 is malfunctioning, and the user can easily make a determination.

As shown in Fig. 7, the first spectrum measurement data 711 and the second spectrum measurement data 712 are data measured from the liquid 63 containing the antibody 19 and the liquid 63 containing the aggregate 20, which are separated from the second purified solution 30 by using the HPLC device 57. Therefore, the first spectrum measurement data 711 is data significantly representing the characteristics of the antibody 19, and the second spectrum measurement data 712 is data significantly representing the characteristics of the aggregate 20. Therefore, it is possible to accurately select the specific wave number band of the aggregate 20.

The target component is the aggregate 20 of the antibody 19. The aggregate 20 has a bad effect of causing the side effects on the biopharmaceutical, and causes the decrease in the drug efficacy of the biopharmaceutical. Therefore, by using the target component as the aggregate 20 and predicting the state thereof, it is possible to suppress the decrease in the drug efficacy of the biopharmaceutical.

As shown in Fig. 14, the concentration prediction model 96LD is the machine learning model, such as the neural network 105. The machine learning model is generally used to predict unknown parameters, and the prediction accuracy can be increased to a certain level through learning. Therefore, the concentration of the aggregate 20 can be predicted with higher accuracy than a linear model, such as a PLS model.

The biopharmaceutical containing the antibody 19 as the target protein, which is called an antibody pharmaceutical, is widely used for the treatment of rare diseases such as hemophilia and Crohn's disease in addition to the treatment of chronic diseases such as cancer, diabetes, and rheumatoid arthritis. Therefore, in a case in which the antibody 19 is used as the target protein, it is possible to promote the development of antibody pharmaceutical widely used for the treatment of various diseases.

The Raman spectrum easily reflects information derived from a functional group of the amino acid of the protein. Therefore, by using the spectrum as the Raman spectrum, the prediction accuracy of the concentration of the aggregate 20, which is the protein, can be further increased.

As shown in Figs. 11 and 12, the specific wave number band is in a range of 1220 cm⁻¹ to 1260 cm⁻¹ and a range of 1650 cm⁻¹ to 1690 cm⁻¹. The range of 1220 cm⁻¹ to 1260 cm⁻¹ is a range in which a band of a so-called amide III attributed to an amide bond of the protein appears. In addition, the range of the wave numbers of 1650 cm⁻¹ to 1690 cm⁻¹ is a range in which a band of an amide I appears. Therefore, it is possible to select the specific wave number band having high validity. It should be noted that the specific wave number band need only be in at least one of a range of 1220 cm⁻¹ to 1260 cm⁻¹ or a range of 1650 cm⁻¹ to 1690 cm⁻1

### [Second Embodiment]

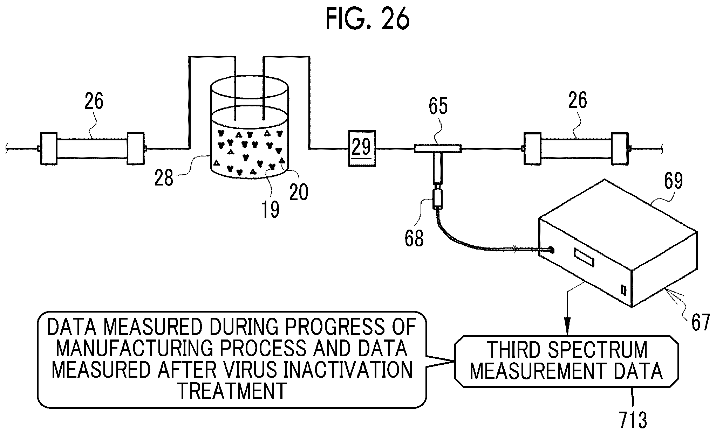

In the first embodiment, the third spectrum measurement data 713 is data measured after the cation chromatography treatment, but the present disclosed technology is not limited to this. As an example, as shown in Fig. 26, the third spectrum measurement data 713 may be data obtained by measuring the Raman spectrum of the first purified solution 28 after the virus inactivation treatment 29 is performed. In this case, the first purified solution 28 is an example of a "suspension" according to the present disclosed technology.

The first purified solution 28 has a closer composition to the culture supernatant solution 17 than the second purified solution 30. Therefore, in a case in which the third spectrum measurement data 713 is data obtained by measuring the Raman spectrum of the first purified solution 28 after the virus inactivation treatment 29 is performed, it can be concluded that the cause of the concentration prediction result 115 being worse than the target value is in the culture conditions of the antibody producing cell 15, and the user can easily make a determination.

The third spectrum measurement data 713 may be data obtained by measuring the Raman spectrum of the third purified solution 31 after an anion chromatography treatment, which is output from the anion chromatography device 27. In addition, the third spectrum measurement data 713 need not be data measured during the progress of the manufacturing process. The first purified solution 28 or the second purified solution 30 may be fractionated and placed on the Raman spectrometer 67 prepared at a different location from the purification line, to measure the third spectrum measurement data 713.

Hereinafter, Example and Comparative Example of the present disclosed technology will be described.

In Example, as described in the first embodiment, first, the culture supernatant solution 17 of the antibody producing cell 15 that produces the antibody 19, in which the antibody gene 14 was incorporated into the cell 13 such as the CHO cell, was generated. Then, the culture supernatant solution 17 was introduced into the immunoaffinity chromatography device 25 and purified, to acquire first purified solution 28. Next, the pretreatment 55 was performed on the first purified solution 28 under the conditions shown in Table 56, to promote the generation of the aggregate 20. Thereafter, the first purified solution 28 was injected into the HPLC device 57 through the autosampler 60, the chromatogram data 64 was measured by the UV detector 62, the Raman spectrum of the first purified solution 28 was measured by using the flow cell 65 and the Raman spectrometer 67, and the spectrum measurement data group 71G was acquired.

The retention time Tan of the antibody 19 and the retention time Tag of the aggregate 20 were derived from the chromatogram data 64, and thus the first spectrum measurement data 711 and the second spectrum measurement data 712 were specified from the spectrum measurement data group 71G. The specific wave number band of the aggregate 20 was selected based on the first spectrum measurement data 711 and the second spectrum measurement data 712.

Next, the culture supernatant solution 17 of the antibody producing cell 15 that produces the antibody 19 was generated in the same manner as described above, the generated culture supernatant solution 17 was introduced into the immunoaffinity chromatography device 25 and the cation chromatography device 26 and purified, to acquire the second purified solution 30. In this case, the Raman spectrum of the second purified solution 30 was measured by using the flow cell 65 and the Raman spectrometer 67 to acquire the spectrum measurement data 71LV, and the aggregate amount 112 was measured by the HPLC device 57, to acquire a total of nine data sets 95.

The cross-validation of the concentration prediction model 96 configured by the neural network 105 was performed by using the total of nine data sets 95 obtained. Specifically, eight of the nine data sets 95 were used as the data sets for training 95L, one was used as the data set for validation 95V, and nine times of the cross-validation were performed while changing the configurations of the data set for training 95L and the data set for validation 95V.

Next, during the progress of the manufacturing process 2, the Raman spectrum of the second purified solution 30 after the cation chromatography treatment was measured by using the flow cell 65 and the Raman spectrometer 67, to acquire third spectrum measurement data 713. Then, the input data 130 composed of only the intensity value of the specific wave number band of the aggregate 20 in the third spectrum measurement data 713 was input to the concentration prediction model 96LD generated by the cross-validation, to output the concentration prediction result 115.

Comparative Example 1 is an example in which the input data 130 of the concentration prediction model 96LD was not limited to the intensity value of the specific wave number band of the aggregate 20, and the intensity values of all the wave number bands of 700 cm⁻¹ to 1800 cm⁻¹ were used. Comparative Example 2 is an example in which the input data 130 of the concentration prediction model 96LD was set to the intensity value of the wave number band selected by the sparse modeling.

Comparative Example 3 is an example in which the concentration prediction model 96LD was a PLS model instead of the neural network 105 as in JP2016-128822A, and the input data 130 of the concentration prediction model 96LD was the intensity value in the wave number band of 800 cm⁻¹ to 1700 cm⁻¹ as in JP2016-128822A. Comparative Example 4 is an example in which the input data 130 of the concentration prediction model 96LD was the intensity value of the wave number band excluding the specific wave number band of the aggregate 20.

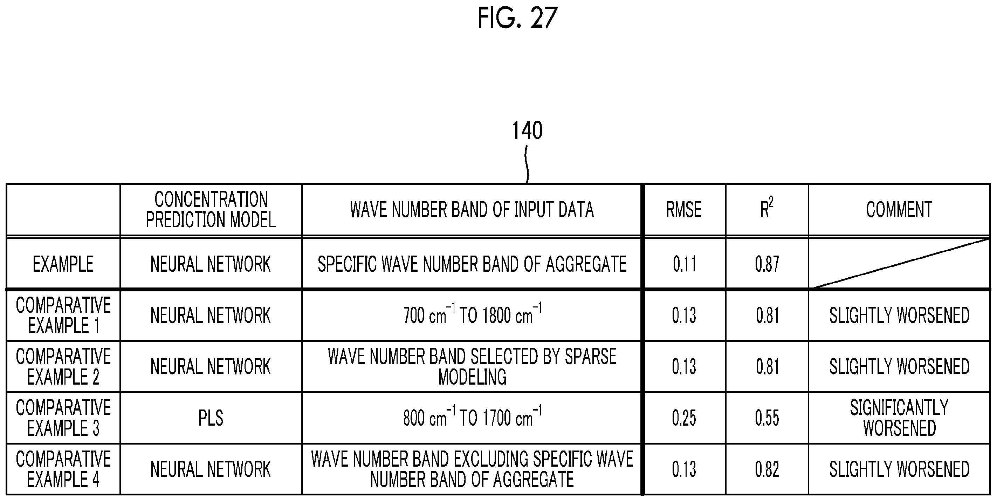

As shown in Table 140 of Fig. 27 as an example, a root-mean-square error (RMSE) of the concentration prediction model 96LD in Example was 0.11, and the coefficient of determination (R²) was 0.87. In contrast, in a case of Comparative Example 1, the RMSE was 0.13 and R² was 0.81, and the prediction accuracy of the concentration prediction model 96LD was slightly worsened as compared with Example. From this result, it was confirmed that the prediction accuracy of the concentration prediction model 96LD was increased by selecting the specific wave number band of the aggregate 20 and setting the input data 130 of the concentration prediction model 96LD to the intensity value of the specific wave number band of the aggregate 20.

Here, since Comparative Example 1 shows the RMSE and the R² that were not inferior to those of Example, it is construed that the prediction accuracy of the concentration prediction model 96LD is good at a glance. However, it cannot be denied that there is a concern that a wave number band irrelevant to the aggregate 20 is perceived to contribute to the prediction of the concentration of the aggregate 20, that is, there is a concern that pseudo correlation occurs. Therefore, it cannot be said that the concentration prediction model 96LD of Comparative Example 1 is reasonable as the model that predicts the concentration of the aggregate 20.

In addition, in a case of Comparative Example 2, the RMSE was 0.13 and R² was 0.81, and the prediction accuracy of the concentration prediction model 96LD was slightly worsened as compared with Example. From this result, it was confirmed that the prediction accuracy of the concentration prediction model 96LD was increased by setting the input data 130 of the concentration prediction model 96LD to the intensity value of the specific wave number band of the aggregate 20, rather than setting the input data 130 of the concentration prediction model 96LD to the intensity value of the wave number band selected by the sparse modeling.

In a case of Comparative Example 3, the RMSE was 0.25, and R² was 0.55, and the prediction accuracy of the concentration prediction model 96LD was significantly worsened as compared with Example. From this result, it was confirmed that the prediction accuracy of the concentration prediction model 96LD was higher than the technology disclosed in JP2016-128822A by configuring the concentration prediction model 96LD with the neural network 105 instead of the PLS model and setting the input data 130 of the concentration prediction model 96LD as the intensity value of the specific wave number band of the aggregate 20.

In addition, in a case of Comparative Example 4, the RMSE was 0.13 and R² was 0.82, and the prediction accuracy of the concentration prediction model 96LD was slightly worsened as compared with Example. From this result, it was confirmed that the prediction accuracy of the concentration prediction model 96LD is increased by using the input data 130 of the concentration prediction model 96LD as the intensity value of the specific wave number band of the aggregate 20. Further, the rationality of the concentration prediction model 96LD generated based on the intensity value of the specific wave number band of the aggregate 20 is also shown.

It should be noted that the target protein is not limited to the antibody 19. A cytokine, a hormone, or the like may be used. The target component is not limited to the aggregate 20. A cell-derived protein, a cell-derived DNA, or the like may be used as the target component.

The spectrum is not limited to the Raman spectrum. An infrared absorption spectrum, a near-infrared absorption spectrum, a nuclear magnetic resonance spectrum, an ultraviolet visible absorption spectroscopy (UV-Vis) spectrum, or a fluorescence spectrum may be used. In a case of the ultraviolet visible absorption spectroscopy spectrum and the fluorescence spectrum, the specific wavelength band is selected instead of the specific wave number band.

The concentration prediction model 96LD may be trained using the data set 95 even after being downloaded to the operation device 41C.

Although the neural network 105 is described as the concentration prediction model 96LD, the present disclosed technology is not limited to this. The neural network 105 may be a decision tree, a random forest, a naive Bayes, a gradient boosting decision tree, or the like.

The concentration prediction model 96LD is not limited to the machine learning model. A model generated by multivariate analysis or statistical analysis may be used. Examples of the multivariate analysis and the statistical analysis include a PLS disclosed in JP2016-128822A, multiple regression, principal component regression, logistic regression, Lasso regression, ridge regression, support vector regression, and Gaussian process regression. In a model generated by such multivariate analysis and statistical analysis, determining a coefficient of a regression equation based on at least two data sets 95 corresponds to "generating the state prediction model using the data set" according to the present disclosed technology.

It should be noted that the state of the target component is not limited to the concentration. For example, the density of the target component may be used. Alternatively, two or more states, such as the concentration and the density, may be predicted.

In each of the above-described embodiments, an example has been described in which the functions of the selection device 41A, the learning device 41B, and the operation device 41C are carried out by three computers, but the present disclosed technology is not limited to this. The functions of the selection device 41A, the learning device 41B, and the operation device 41C may be implemented by one computer. In addition, the function of the selection device 41Amay be implemented by one computer, and the functions of the learning device 41B and the operation device 41C may be implemented by one computer. The functions of the selection device 41A, the learning device 41B, and the operation device 41C may be shared among four or more computers. As described above, the information processing device according to the present disclosure may be carried out by one computer, or may be carried out by a plurality of computers.

In each of the above-described embodiments, for example, as a hardware structure of processing units that execute various types of processing, such as the acquisition units 80 and 120, the RW control units 81, 100, and 121, the selection unit 82, the training validation unit 101, the prediction unit 122, and the display control unit 123, various processors shown below can be used. As described above, the various processors include, in addition to the CPUs 47A to 47C, which are general-purpose processors that execute software (operation programs 75A to 75C) to function as the various processing units, a programmable logic device (PLD), which is a processor of which a circuit configuration can be changed after the manufacturing, such as a field programmable gate array (FPGA), a dedicated electric circuit, which is a processor having a circuit configuration designed exclusively for executing specific processing, such as an application specific integrated circuit (ASIC), and the like.

One processing unit may be configured by one of these various processors, or may be configured by a combination of two or more processors of the same type or different types (for example, a combination of a plurality of FPGAs and/or a combination of a CPU and an FPGA). In addition, a plurality of the processing units may be configured by one processor.

As an example in which the plurality of processing units are configured by one processor, first, as represented by a computer, such as a client and a server, there is a form in which one processor is configured by a combination of one or more CPUs and software, and the processor functions as the plurality of processing units. Second, as represented by a system on a chip (SoC) or the like, there is a form in which a processor, which implements the functions of the entire system including the plurality of processing units with a single integrated circuit (IC) chip, is used. In this manner, as the hardware structure, the various processing units are configured by using one or more of the various processors described above.

Further, as the hardware structure of the various processors, more specifically, an electric circuit (circuitry) in which circuit elements such as semiconductor elements are combined can be used.

Based on the above description, the technologies according to the following supplementary notes can be understood.

### [Supplementary Note 1]

An information processing device comprising: a processor, in which the processor is configured to: as preparatory processing for generating a state prediction model that predicts a state of a target component in a suspension produced in a manufacturing process of a biopharmaceutical containing a target protein as an active ingredient, acquire first spectrum measurement data obtained by measuring a spectrum of an electromagnetic wave emitted from the target protein and second spectrum measurement data obtained by measuring a spectrum of an electromagnetic wave emitted from the target component; and select a specific wave number band or a specific wavelength band that is specific to the target component by comparing an intensity value of the first spectrum measurement data and an intensity value of the second spectrum measurement data.

### [Supplementary Note 2]

The information processing device according to supplementary note 1, in which the state prediction model is generated by using a data set including an intensity value of the specific wave number band or the specific wavelength band and ground truth data of the state of the target component.

### [Supplementary Note 3]

The information processing device according to supplementary note 2, in which the state of the target component is a concentration of the target component in the suspension, and a concentration of the target protein and the concentration of the target component in the suspension as a source of the data set are in a range of 0.001 mg/mL to 20 mg/mL.

### [Supplementary Note 4]

The information processing device according to any one of supplementary notes 1 to 3, in which a suspension to be used to select the specific wave number band or the specific wavelength band is subjected to a pretreatment for promoting generation of the target component.

### [Supplementary Note 5]

The information processing device according to any one of supplementary notes 1 to 4, in which the state prediction model outputs a prediction result of the state of the target component in accordance with an intensity value of the specific wave number band or the specific wavelength band in third spectrum measurement data obtained by measuring a spectrum of an electromagnetic wave emitted from a suspension in which the state of the target component is unknown.

### [Supplementary Note 6]

The information processing device according to supplementary note 5, in which the third spectrum measurement data is data measured during progress of the manufacturing process.

### [Supplementary Note 7]

The information processing device according to supplementary note 5 or 6, in which the third spectrum measurement data is data measured after a virus inactivation treatment or after a cation chromatography treatment.

### [Supplementary Note 8]

The information processing device according to any one of supplementary notes 1 to 7, in which the first spectrum measurement data and the second spectrum measurement data are data measured from a first solution containing the target protein and a second solution containing the target component, the first solution and the second solution being separated from the suspension by using a high-performance liquid chromatography device.

### [Supplementary Note 9]

The information processing device according to any one of supplementary notes 1 to 8, in which the target component is an aggregate of the target protein.

### [Supplementary Note 10]

The information processing device according to any one of supplementary notes 1 to 9, in which the state prediction model is a machine learning model.

### [Supplementary Note 11]

The information processing device according to any one of supplementary notes 1 to 10, in which the target protein is an antibody.

### [Supplementary Note 12]

The information processing device according to any one of supplementary notes 1 to 11, in which the spectrum is a Raman spectrum.

### [Supplementary Note 13]

The information processing device according to supplementary note 12, in which the specific wave number band is in at least any one of a range of 1220 cm⁻¹ to 1260 cm⁻¹ or a range of 1650 cm⁻¹ to 1690 cm⁻¹.

The present disclosed technology can also be combined with various embodiments and/or various modification examples described above, as appropriate. In addition, it goes without saying that the present disclosure is not limited to each of the embodiments described above, various configurations can be adopted as long as the configuration does not deviate from the gist. Further, the present disclosed technology includes, in addition to the program, a storage medium that stores the program in a non-transitory manner.

The above-described contents and the above-shown contents are the detailed description of the parts according to the present disclosed technology, and are merely an example of the present disclosed technology. For example, the above description of the configuration, the function, the operation, and the effect are the description of examples of the configuration, the function, the operation, and the effect of the parts according to the present disclosed technology. Accordingly, it goes without saying that unnecessary parts may be deleted, new elements may be added, or replacements may be made with respect to the above-described contents and the above-shown contents within a range that does not deviate from the gist of the present disclosed technology. In addition, in order to avoid complications and facilitate grasping the parts according to the present disclosed technology, in the above-described contents and the above-shown contents, the description of technical general knowledge and the like that do not particularly require description for enabling the implementation of the present disclosed technology are omitted.

In the present specification, "A and/or B" is synonymous with "at least one of A or B". That is, "A and/or B" means that it may be only A, only B, or a combination of A and B. In addition, in the present specification, also in a case in which three or more matters are expressed in association by "and/or", the same concept as "A and/or B" is applied.

All of the documents, the patent applications, and the technical standards described in the present specification are incorporated herein by reference to the same extent as in a case in which each of the documents, patent applications, and technical standards is specifically and individually described by being incorporated by reference.

## Claims

1. An information processing device comprising:
a processor,
wherein the processor is configured to:
as preparatory processing for generating a state prediction model that predicts a state of a target component in a suspension produced in a manufacturing process of a biopharmaceutical containing a target protein as an active ingredient,
acquire first spectrum measurement data obtained by measuring a spectrum of an electromagnetic wave emitted from the target protein and second spectrum measurement data obtained by measuring a spectrum of an electromagnetic wave emitted from the target component; and
select a specific wave number band or a specific wavelength band that is specific to the target component by comparing an intensity value of the first spectrum measurement data and an intensity value of the second spectrum measurement data.

2. The information processing device according to claim 1,
wherein the state prediction model is generated by using a data set including an intensity value of the specific wave number band or the specific wavelength band and ground truth data of the state of the target component.

3. The information processing device according to claim 2,
wherein the state of the target component is a concentration of the target component in the suspension, and
a concentration of the target protein and the concentration of the target component in the suspension as a source of the data set are in a range of 0.001 mg/mL to 20 mg/mL.

4. The information processing device according to claim 1,
wherein a suspension to be used to select the specific wave number band or the specific wavelength band is subjected to a pretreatment for promoting generation of the target component.

5. The information processing device according to claim 1,
wherein the state prediction model outputs a prediction result of the state of the target component in accordance with an intensity value of the specific wave number band or the specific wavelength band in third spectrum measurement data obtained by measuring a spectrum of an electromagnetic wave emitted from a suspension in which the state of the target component is unknown.

6. The information processing device according to claim 5,
wherein the third spectrum measurement data is data measured during progress of the manufacturing process.

7. The information processing device according to claim 5,
wherein the third spectrum measurement data is data measured after a virus inactivation treatment or after a cation chromatography treatment.

8. The information processing device according to claim 1,
wherein the first spectrum measurement data and the second spectrum measurement data are data measured from a first solution containing the target protein and a second solution containing the target component, the first solution and the second solution being separated from the suspension by using a high-performance liquid chromatography device.

9. The information processing device according to claim 1,
wherein the target component is an aggregate of the target protein.

10. The information processing device according to claim 1,
wherein the state prediction model is a machine learning model.

11. The information processing device according to claim 1,
wherein the target protein is an antibody.

12. The information processing device according to claim 1,
wherein the spectrum is a Raman spectrum.

13. The information processing device according to claim 12,
wherein the specific wave number band is in at least any one of a range of 1220 cm⁻¹ to 1260 cm⁻¹ or a range of 1650 cm⁻¹ to 1690 cm⁻¹.

14. An operation method of an information processing device, the operation method comprising:
as preparatory processing for generating a state prediction model that predicts a state of a target component in a suspension produced in a manufacturing process of a biopharmaceutical containing a target protein as an active ingredient,
acquiring first spectrum measurement data obtained by measuring a spectrum of an electromagnetic wave emitted from the target protein and second spectrum measurement data obtained by measuring a spectrum of an electromagnetic wave emitted from the target component; and
selecting a specific wave number band or a specific wavelength band that is specific to the target component by comparing an intensity value of the first spectrum measurement data and an intensity value of the second spectrum measurement data.

15. An operation program of an information processing device, the operation program causing a computer to execute a process comprising:
as preparatory processing for generating a state prediction model that predicts a state of a target component in a suspension produced in a manufacturing process of a biopharmaceutical containing a target protein as an active ingredient,
acquiring first spectrum measurement data obtained by measuring a spectrum of an electromagnetic wave emitted from the target protein and second spectrum measurement data obtained by measuring a spectrum of an electromagnetic wave emitted from the target component; and
selecting a specific wave number band or a specific wavelength band that is specific to the target component by comparing an intensity value of the first spectrum measurement data and an intensity value of the second spectrum measurement data.

16. A state prediction model causing a computer to execute a function comprising:
outputting a prediction result of a state of a target component in a suspension produced in a manufacturing process of a biopharmaceutical containing a target protein as an active ingredient, in accordance with an intensity value of a wave number band or a wavelength band that is specific to the target component among intensity values of wave numbers or wavelengths of spectrum measurement data obtained by measuring a spectrum of an electromagnetic wave emitted from the suspension.
